# EUROPEAN PATENT APPLICATION

(11) **EP 1 172 106 A2**
(43) Date of publication of application: **16.01.2002**
(21) Application number: 01303983.9
(22) Date of filing: 01.05.2001
(51) Int. Cl.: A61K 31/445, A61K 31/435

(54) **Use of fluoroalkoxybenzylamino derivatives of nitrogen containing heterocycles as substance P receptor antagonists**

(30) Priority: 03.05.2000 US 201591 P; 04.10.2000 US 237780 P
(71) Applicant: Pfizer Products Inc., Groton, Connecticut 06340 (US)
(72) Inventor: Chappel, Phillip Branch, Pfizer Global, Groton, Connecticut 06340 (US); O'Neill, Brian Thomas, Pfizer Global, Groton, Connecticut 06340 (US); Saltarelli, Mario David, Pfizer Global, Groton, Connecticut 06340 (US)
(74) Representative: Motion, Keith Robert

(57) **Abstract**

The present invention relates to methods of treating various CNS and other disorders by adminstering fluoroalkoxybenzylamino derivatives of nitrogen containing heterocyclic compounds, and specifically, by administering compounds of the formula 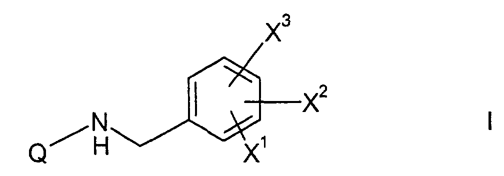 wherein Q, X¹, X² and X³ are as defined below, and their pharmaceutically acceptable salts.

## Description

### Background of the Invention

The present invention relates to methods of treating various CNS and other disorders by administering fluoroalkoxybenzylamino derivatives of nitrogen containing heterocycles. The fluoroalkoxybenzyl derivatives that are used in the novel methods of this invention exhibit pharmaceutical activity as substance P receptor antagonists.

Substance P is a naturally occurring undecapeptide belonging to the tachykinin family of peptides, the latter being named because of their prompt stimulatory action on smooth muscle tissue. More specifically, substance P is a pharmacologically active neuropeptide that is produced in mammals (having originally been isolated from gut) and possesses a characteristic amino acid sequence that is illustrated by D. F. Veber et al. in U.S. Patent No. 4,680,283. The wide involvement of substance P and other tachykinins in the pathophysiology of numerous diseases has been amply demonstrated in the art.

Piperidine derivatives, quinuclidine derivatives and related heterocyclic nitrogen containing compounds that are useful as substance P antagonists are referred to in United States Patent 5,733,450, which was issued on June 30, 1998; United States Patent 5,232,929, which was issued on August 3, 1993; United States Patent 5,162,339, which was issued on November 10, 1992; United States Patent 5,451,586, which issued on September 19, 1995; and European Patent 550,635, which was granted on August 20, 1991. All of the foregoing patents are incorporated herein by reference in their entireties.

### Summary of the Invention

The present invention relates to a method of treating a disorder or condition selected from sleep disorders (e.g., sleep apnea, insomnia, somnambulism, sleep deprivation, REM sleep disorders, hypersomnia, parasomnias, sleep-wake cycle disorders, narcolepsy, sleep disorders associated with shift work or irregular work schedules, and other sleep disorders); autism; pervasive development disorder; rheumatoid arthritis; osteoarthritis; fibromyalgia; human immunodeficiency virus (HIV) infections; dissociative disorders such as body dysmorphic disorders; eating disorder such as anorexia and bulimia; ulcerative colitis; Crohn's disease; irritable bowel syndrome; functional abdominal pain; chronic fatigue syndrome; sudden infant death syndrome (SIDS); overactive bladder; chronic cystitis; chemotherapy induced cystitis; cough, angiotensin converting enzyme (ACE) induced cough; itch; hiccups; premenstrual syndrome: premenstrual dysphoric disorder; schizophrenia; schizoaffective disorder; delusional disorder; substance-induced psychotic disorder; brief psychotic disorder; shared psychotic disorder; psychotic disorder due to a general medical condition; schizophreniform disorder; amenorrheic disorders such as desmenorrhea; obesity; epilepsy: movement disorders such as primary movement disorders, spasticities, Scott's syndrome, Tourette's syndrome, palsys (e.g., Bell's palsy, cerebral palsy, birth palsy, brachial palsy, wasting palsy, ischemic palsy, progressive bulbar palsy and other palsys), amyolateral sclerosis (ALS), akinetic-rigid disorders, akinesias, dyskinesias (*e*.*g*., familial paroxysmal dyskinesia, tardive dyskinesia, tremor, chorea, myoclonus, tics and other dyskinesias) restless leg syndrome and movement disorders associated with Parkinson's disease or Huntington's disease; mastalgia syndromes; motion sickness; immune dysfunctions *(e.g.,* stress induced immune dysfunctions such as idiopathic immune dysfunctions, post infection immune dysfunctions, post lumpectomy immune dysfunctions, porcine stress syndrome, bovine shipping fever, equine paroxysmal fibrillation, confinement dysfunction in chicken, sheering stress in sheep, and human-animal interaction stress in dogs); generalized anxiety disorder; panic disorder; phobias, including social phobia, agoraphobia, and specific phobias; obsessive-compulsive disorder; post-traumatic stress disorder; depression including major depressive disorder, single episode depression, recurrent depression, child abuse induced depression, postpartum depression and dysthemia; cyclothymia; bipolar disorder; neurocardiac disorders such as neurocardiac syncope, neurogenic syncope, hypersensitive Carotid sinus, neurovascular syndrome and arrythmias including arrythmias secondary to gastrointestinal disturbances; addiction disorders involving addictions to behaviors (*e*.*g*., addictions to gambling and other addictive behaviors); HIV-1 associated dementia; AIDS dementia complex (ADC); HIV encephalopathy; HIV related neuralgias; AIDS related neuralgias; epilepsy; and attention deficit hyperactivity disorder in a mammal, including a human, comprising administering to said mammal an amount of a compound of the formula I, 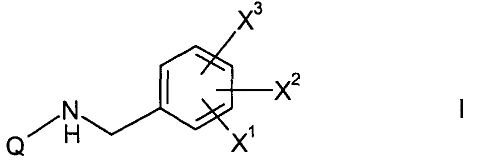 wherein X¹ is hydrogen, (C₁-C₁₀) alkoxy optionally substituted with from one to three flourine atoms or (C₁-C₁₀) alkyl optionally substituted with from one to three fluorine atoms;

X² and X³ are independently selected from hydrogen, halo, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, -C(=O)-NH-(C₁-C₆)alkyl, (C₁-C₆) alkyl-C(=O)-NH-(C₁-C₆) alkyl, hydroxy(C₁-C₄)alkyl, (C₁-C₄)alkoxy(C₁-C₄)alkyl, -NHC(=O)H and -NHC(=O)-(C₁-C₆) alkyl; and Q is a group of the formula 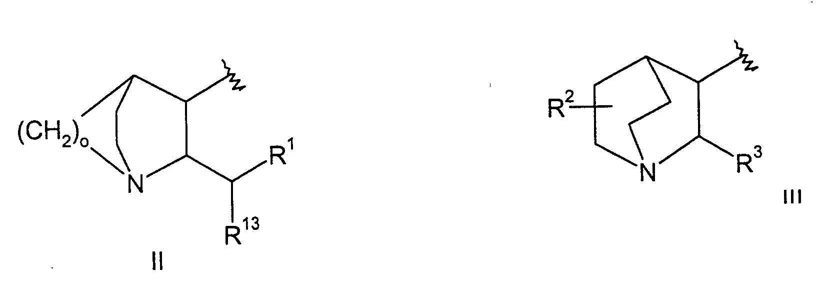 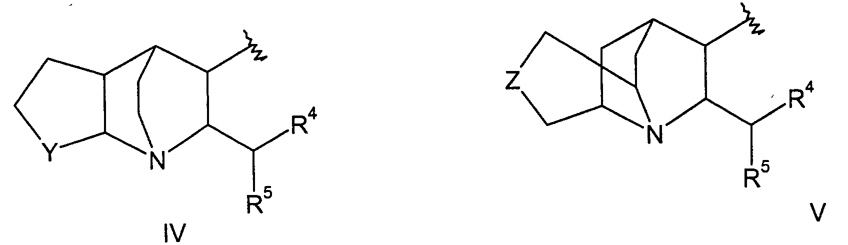 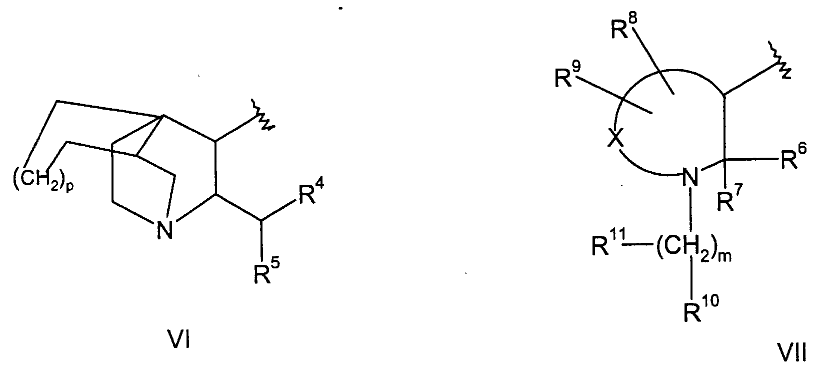 OR 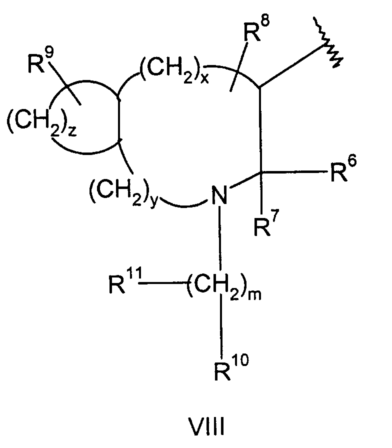 wherein R¹ is a radical selected from furyl, thienyl, pyridyl, indolyl, biphenyl and phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R¹³ is selected from (C₃-C₄) branched alkyl, (C₅-C₆) branched alkenyl, (C₅-C₇) cycloalkyl, and the radicals named in the definition of R¹;
R² is hydrogen or (C₁-C₆) alkyl;
R³ is phenyl, biphenyl, naphthyl, pyridyl, benzhydryl, thienyl or furyl, and R³ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)₁ wherein I is an integer from one to three, or Y is a group of the formula 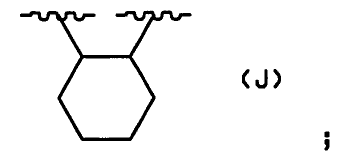
Z is oxygen, sulfur, amino, (C₁-C₃)alkylamino or (CH₂)ₙ wherein n is zero, one or two;
o is two or three;
p is zero or one;
x is an integer from zero to four;
y is an integer from zero to four;
z is an integer from one to six, and the ring in formula VIII containing (CH₂)_{z} may contain from zero to three double bonds, and one of the carbons of said (CH₂)_{z} may optionally be replaced by oxygen, sulphur or nitrogen;
R⁴ is furyl, thienyl, pyridyl, indolyl, biphenyl, or phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, (C₁-C₃) alkoxy-carbonyl and benzyloxycarbonyl;
R⁵ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R⁸, and wherein any one of the carbon atoms of said (CH₂)_{q} may optionally be substituted with R⁹;
m is an integer from 0 to 8, and any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R¹¹;
R⁶ is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆) alkyl-O-C(=O)- (C₁-C₆)alkyl, (C₁-C₆)alkyl-C(=O)-O-, (C₁-C₆)alkyl-C(=O)- (C₁-C₆)alkyl-O-, (C₁-C₆)alkyl-C(=O)-, (C₁-C₆)alkyl-C(=O)- (C₁-C₆)alkyl-, di-(C₁-C₆)alkylamino, -C(=O)NH-(C₁-C₆)alkyl,(C₁-C₆)-alkyl-C(=O)-NH-(C₁-C₆)alkyl, -NHC(=O)H and-NHC(=O)-(C₁-C₆) alkyl; and wherein one of the phenyl moieties of said benzhydryl may optionally be replaced by naphthyl, thienyl, furyl or pyridyl;
R⁷ is hydrogen, phenyl or (C₁-C₆)alkyl;
or R⁶ and R⁷, together with the carbon to which they are attached, form a saturated carbocyclic ring having from 3 to 7 carbon atoms wherein one of said carbon atoms may optionally be replaced by oxygen, nitrogen or sulfur;
R⁸ and R⁹ are each independently selected from hydrogen, hydroxy, halo, amino, oxo (=O), nitrite, hydroxy-(C₁-C₆)alkyl, (C₁-C₆)alkoxy-(C₁-C₆)alkyl, (C₁-C₆)alkylamino, di-(C₁-C₆)alkylamino, (C₁-C₆)alkoxy, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C(=O)-, (C₁-C₆)alkyl-C(=O)-(C₁-C₆)alkyl-, and the radicals set forth in the definition of R⁶;
R¹⁰ is NHCR¹², NHCH₂R¹², NHSO₂R¹² or one of the radicals set forth in any of the definitions of R⁶, R⁸ and R⁹;
R¹¹ is oximino (=NOH) or one of the radicals set forth in any of the definitions of R⁶, R⁸ and R⁹; and
R¹² is (C₁-C₆)alkyl, hydrogen, phenyl(C₁-C₆)alkyl or phenyl optionally substituted with (C₁-C₆) alkyl; and
with the proviso that (a) when m is 0, R¹¹ is absent, (b) neither R⁸, R⁹, R¹⁰ nor R¹¹ can form, together with the carbon to which it is attached, a ring with R⁷, (c) when Q is a group of the formula VIII, R⁸ and R⁹ cannot be attached to the same carbon atom, (d) when R⁸ and R⁹ are attached to the same carbon atom, then either each of R⁸ and R⁹ is independently selected from hydrogen, fluoro, (C₁-C₆) alkyl, hydroxy-(C₁-C₆)alkyl and (C₁-C₆)alkoxy-(C₁-C₆)alkyl, or R⁸ and R⁹, together with the carbon to which they are attached, form a (C₃-C₆) saturated carbocyclic ring that forms a spiro compound with the nitrogen-containing ring to which they are attached, (e) when neither X¹, X² nor X³ is a fluorinated alkoxy group, at least one of R¹, R³, R⁴, R⁵, R⁶, R⁷ and R¹³ is an aryl group substituted with a fluorinated alkoxy group;
or a pharmaceutically acceptable salt thereof,
or a compound selected from the group consisting of (and hereinafter referred to, collectively, as "the Group A compounds"):
   (2S,3S)-3-(6-methoxy-3-trifluoromethyl-1,3-dihydroisobenzofuran-5-yl)methylamino-2-phenylpiperidine;
   (2S,3S)-3-(6-methoxy-1-methyl-1-trifluoromethylisochroman-7-yl)methylamino-2-phenylpiperidine;
   (2S,3S)-3-(6-methoxy-3-methyl-3-trifluoromethyl-1,3-dihydroisobenzofuran-5-yl)methylamino-2-phenylpiperidine;
   (2S,3S)-3-(6-methoxy-3-phenyl-3-trifluoromethyl-1,3-dihydroisobenzofuran-5-yl)methylamino-2-phenylpiperidine;
   (2S,3S)-3-[1-(6-methoxy-3-methyl-3-trifluoromethyl-1,3-dihydroisobenzofuran-5-yl)ethylamino]-2-phenylpiperidine;
   (2S,3S)-3-[(1R)-6-methoxy-1-methyl-1-trifluoromethylisochroman-7-yl]methylamino-2-phenylpiperidine;
   (2S,3S)-3-[(3R)-6-methoxy-3-methyl-3-trifluoromethyl-1,3-dihydroisobenzofuran-5-yl)methylamino-2-phenylpiperidine;
   (2S,3S)-N-(5-ethyl-2-methoxyphenyl)methyl-2-diphenylmethyl-1-azabi-cyclo[2.2.2]-octan-3-amine;
   (2S,3S)-N-(5-isopropyl-2-methoxyphenyl)methyl-2-di-phenylmethyl-1-azabicyclo[2.2.2]-octan-3-amine;
   (2S,3S)-N-(5-sec-butyl-2-methoxyphenyl)-methyl-2-diphenylmethyl-1-azabicyclo[2.2.2]-octan-3-amine;
   (2S,3S)-N-(5-tert-butyl-2-methoxyphenyl)-methyl-2-diphenylmethyl-1-azabicyclo[2.2.2]-octan-3-amine; and
   (2S,3S)-N-(5-methyl-2-methoxyphenyl)methyl-2-diphenylmethyl-1-azabicyclo[2.2.2]-octan-3-amine;
   or a pharmaceutically acceptable salt thereof, that is effective in treating such disorder or condition.

The term "treating", as used herein, refers to, and includes, reversing, alleviating, inhibiting the progress of, or preventing a disease, disorder or condition, or one or more symptoms thereof; and the term "treatment", as used herein, refers to the act of treating, where "treating" is defined as above.

The term "halo", as used herein, unless otherwise indicated, includes chloro, fluoro, bromo and iodo.

The term "alkyl", as used herein, unless otherwise indicated, includes saturated monovalent hydrocarbon radicals having straight, branched or cyclic moieties or combinations thereof.

The term "one or more substituents," as used herein, includes from one to the maximum number of substituents possible based on the number of available bonding sites.

The present invention also relates to a method of treating a disorder or condition selected from the group consisting of sleep disorders (*e*.*g*., sleep apnea, insomnia, somnambulism, sleep deprivation, REM sleep disorders, hypersomnia, parasomnias, sleep-wake cycle disorders, narcolepsy, sleep disorders associated with shift work or irregular work schedules, and other sleep disorders); pervasive development disorder; rheumatoid arthritis; osteoarthritis; fibromyalgia; human immunodeficiency virus (HIV) infections; dissociative disorders such as body dysmorphic disorders; eating disorder such as anorexia and bulimia; ulcerative colitis; Crohn's disease; irritable bowel syndrome; functional abdominal pain; chronic fatigue syndrome; sudden infant death syndrome (SIDS); overactive bladder; chronic cystitis; chemotherapy induced cystitis; cough, angiotensin converting enzyme (ACE) induced cough; itch; hiccups; premenstrual syndrome: premenstrual dysphoric disorder; schizophrenia; schizoaffective disorder; delusional disorder; substance-induced psychotic disorder; brief psychotic disorder; shared psychotic disorder; psychotic disorder due to a general medical condition; schizophreniform disorder; amenorrheic disorders such as desmenorrhea; obesity; epilepsy: movement disorders such as primary movement disorders, spasticities, Scott's syndrome, Tourette's syndrome, palsys (e.g., Bell's palsy, cerebral palsy, birth palsy, brachial palsy, wasting palsy, ischemic palsy, progressive bulbar palsy and other palsys), amyolateral sclerosis (ALS), akinetic-rigid disorders, akinesias, dyskinesias (e.g., familial paroxysmal dyskinesia, tardive dyskinesia, tremor, chorea, myoclonus, tics and other dyskinesias) restless leg syndrome and movement disorders associated with Parkinson's disease or Huntington's disease; mastalgia syndromes; motion sickness; immune dysfunctions (e.g., stress induced immune dysfunctions such as idiopathic immune dysfunctions, post infection immune dysfunctions, post lumpectomy immune dysfunctions, porcine stress syndrome, bovine shipping fever, equine paroxysmal fibrillation, confinement dysfunction in chicken, sheering stress in sheep, and human-animal interaction stress in dogs); generalized anxiety disorder; panic disorder; phobias, including social phobia, agoraphobia, and specific phobias; obsessive-compulsive disorder; post-traumatic stress disorder; depression including major depressive disorder, single episode depression, recurrent depression, child abuse induced depression, postpartum depression and dysthymia; cyclothymia; bipolar disorder; neurocardiac disorders such as neurocardiac syncope, neurogenic syncope, hypersensitive Carotid sinus, neurovascular syndrome and arrythmias including arrythmias secondary to gastrointestinal disturbances; addiction disorders involving addictions to behaviors (*e*.*g*., addictions to gambling and other addictive behaviors); HIV-1 associated dementia; HIV encephalopathy; AIDS dementia complex (ADC); HIV related neuralgias; AIDS related neuralgias; epilepsy; and attention deficit hyperactivity disorder in a mammal, including a human, comprising administering to said mammal an amount of a compound of the formula I or Group A compound, as defined above, or a pharmaceutically acceptable salt thereof, that is effective in antagonizing the effect of substance P at its receptor site.

Other more specific methods of this invention include any of the above methods wherein the disorder or condition that is being treated is selected from movement disorders such as primary movement disorders, spasticities, Scott's syndrome, Tourette's syndrome, palsys (e.g., Bell's palsy, cerebral palsy, birth palsy, brachial palsy, wasting palsy, ischemic palsy, progressive bulbar palsy and other palsys), amyolateral sclerosis (ALS), akinetic-rigid disorders, akinesias, dyskinesias (*e*.*g*., familial paroxysmal dyskinesia, tardive dyskinesia, tremor, chorea, myoclonus, tics and other dyskinesias) restless leg syndrome and movement disorders associated with Parkinson's disease or Huntington's disease.

Other more specific methods of this invention include the above methods wherein the disorder or condition that is being treated is major depressive disorder.

Other more specific methods of this invention include the above methods wherein the disorder or condition that is being treated is major depressive disorder, and wherein the mammal being treated is a human who has not exhibited an adequate treatment response following treatment for the same disorder or condition with a selective serotonin reuptake inhibitor (SSRI). The phrase "adequate treatment response" to an SSRI, as used herein, means that the SSRI with which the human patient was treated in accordance with a treatment protocol accepted by those of skill in the art of treating the disorder or condition for which such patient was being treated did not result in a degree of amelioration of the symptoms of such disorder or condition that would cause such persons of skill in the art to consider such treatment successful.

Other more specific methods of this invention include the above methods wherein the disorder or condition that is being treated is somatic major depressive disorder.

Other more specific methods of this invention include the above methods wherein the disorder or condition that is being treated is somatic major depressive disorder, and wherein the mammal being treated is a human who has not exhibited an adequate treatment response following treatment for the same disorder or condition with a selective serotonin reuptake inhibitor (SSRI). The phrase "adequate treatment response" to an SSRI, as used herein, means that the SSRI with which the human patient was treated in accordance with a treatment protocol accepted by those of skill in the art of treating the disorder or condition for which such patient was being treated did not result in a degree of amelioration of the symptoms of such disorder or condition that would cause such persons of skill in the art to consider such treatment successful.

Other more specific methods of this invention include the above methods wherein the disorder or condition that is being treated is irritable bowel syndrome.

Other more specific methods of this invention include the above methods wherein the disorder or condition that is being treated is an HIV infection.

Other more specific methods of this invention include the above methods wherein the disorder or condition that is being treated is selected from HIV-1 associated dementia, AIDS dementia complex (ADC), HIV encephalopathy, and HIV related neuralgias.

Other more specific methods of this invention include the above methods wherein the disorder or condition that is being treated is selected from immune dysfunctions (e.g., stress induced immune dysfunctions such as idiopathic immune dysfunctions, post infection immune dysfunctions, post lumpectomy immune dysfunctions, porcine stress syndrome, bovine shipping fever, equine paroxysmal fibrillation, confinement dysfunction in chicken, sheering stress in sheep, and human-animal interaction stress in dogs).

Other more specific methods of this invention include the above methods wherein the disorder or condition that is being treated is selected from neurocardiac disorders such as neurocardiac syncope, neurogenic syncope, hypersensitive Carotid sinus, neurovascular syndrome and arrythmias including arrythmias secondary to gastrointestinal disturbances.

Other more specific methods of this invention include the above methods wherein the disorder or condition that is being treated is selected from major depression, single episode depression, recurrent depression, child abuse induced depression, postpartum depression, dysthymia, cyclothymia and bipolar disorder.

Other more specific methods of this invention include the above methods wherein the disorder or condition that is being treated is selected from major depression, single episode depression, recurrent depression, child abuse induced depression, postpartum depression, dysthymia, cyclothymia and bipolar disorder, and wherein the mammal being treated is a human who has not exhibited an adequate treatment response following treatment for the same disorder or condition with a selective serotonin reuptake inhibitor (SSRI). The phrase "adequate treatment response" to an SSRI, as used herein, means that the SSRI with which the human patient was treated in accordance with a treatment protocol accepted by those of skill in the art of treating the disorder or condition for which such patient was being treated did not result in a degree of amelioration of the symptoms of such disorder or condition that would cause such persons of skill in the art to consider such treatment successful.

Other more specific methods of this invention include the above methods wherein the disorder or condition that is being treated is selected from as body dysmorphic disorders and eating disorders such as anorexia and bulimia.

Other more specific methods of this invention include the above methods wherein the disorder or condition that is being treated is selected from schizophrenia, schizoaffective disorder, delusional disorder, substance-induced psychotic disorder, brief psychotic disorder, shared psychotic disorder, psychotic disorder due to a general medical condition, and schizophreniform disorder.

Other more specific methods of this invention include the above methods wherein the disorder or condition that is being treated is selected from premenstrual syndrome, premenstrual dysphoric disorder, and amenorrheic disorders such as desmenorrhea.

Other more specific methods of this invention include the above methods wherein the disorder or condition that is being treated is selected from premenstrual syndrome, premenstrual dysphoric disorder, and amenorrheic disorders such as desmenorrhea, and wherein the mammal being treated is a human who has not exhibited an adequate treatment response following treatment for the same disorder or condition with a selective serotonin reuptake inhibitor (SSRI). The phrase "adequate treatment response" to an SSRI, as used herein, means that the SSRI with which the human patient was treated in accordance with a treatment protocol accepted by those of skill in the art of treating the disorder or condition for which such patient was being treated did not result in a degree of amelioration of the symptoms of such disorder or condition that would cause such persons of skill in the art to consider such treatment successful.

Other more specific methods of this invention include the above methods wherein the disorder or condition that is being treated is selected from Crohn's disease, irritable bowel syndrome and functional abdominal pain.

Other more specific methods of this invention include the above methods wherein the disorder or condition that is being treated is selected from autism, pervasive development disorder, and attention deficit hyperactivity disorder.

Other more specific method of this invention include the above methods wherein the disorder or condition that is being treated is selected from chronic fatigue syndrome, sudden infant death syndrome (SIDS), obesity, and epilepsy.

Other more specific methods of this invention include the above methods wherein the disorder or condition that is being treated is selected from generalized anxiety disorder, panic disorder, obsessive-compulsive disorder, post-traumatic stress disorder, and phobias, including social phobia, agoraphobia, and specific phobias.

Other more specific methods of this invention include the above methods wherein the disorder or condition that is being treated is selected from generalized anxiety disorder, panic disorder, obsessive-compulsive disorder, post-traumatic stress disorder, and phobias, including social phobia, agoraphobia, and specific phobias, and wherein the mammal being treated is a human who has not exhibited an adequate treatment response following treatment for the same disorder or condition with a selective serotonin reuptake inhibitor (SSRI). The phrase "adequate treatment response" to an SSRI, as used herein, means that the SSRI with which the human patient was treated in accordance with a treatment protocol accepted by those of skill in the art of treating the disorder or condition for which such patient was being treated did not result in a degree of amelioration of the symptoms of such disorder or condition that would cause such persons of skill in the art to consider such treatment successful.

Other more specific methods of this invention include the above methods wherein the disorder or condition that is being treated is selected from cough, angiotensin converting enzyme (ACE) induced cough, itch, and hiccups.

Other more specific methods of this invention include the above methods wherein the disorder or condition that is being treated is selected from overactive bladder; chronic cystitis and chemotherapy induced cystitis.

Other more specific methods of this invention include the above methods wherein the disorder or condition that is being treated is attention deficit hyperactivity disorder.

Other more specific methods of this invention include the above methods wherein the disorder or condition that is being treated is selected from sleep disorders (e.g., sleep apnea, insomnia, somnambulism, sleep deprivation, REM sleep disorders, hypersomnia, parasomnias, sleep-wake cycle disorders, narcolepsy, sleep disorders associated with shift work or irregular work schedules, and other sleep disorders).

The present invention also relates to a method of treating a disorder or condition selected from the group consisting of pain resulting from soft tissue and peripheral damage, such as acute trauma; postherpetic neuralgia, trigeminal neuralgia, segmental or intercostal neuralgia and other neuralgias; pain associated with osteoarthritis and rheumatoid arthritis; musculo-skeletal pain, such as pain experienced after trauma; spinal pain, dental pain, myofascial pain syndromes, episiotomy pain, and pain resulting from burns; deep and visceral pain, such as heart pain, muscle pain, eye pain, orofacial pain, for example, odontalgia, abdominal pain, gynaecological pain, for example, dysmenorrhoea, labour pain and pain associated with endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, neuropathic lower back pain, HIV related neuropathic pain, diabetic neuropathic pain, and arachnoiditis; neuropathic and non-neuropathic pain associated with carcinoma, often referred to as cancer pain; central nervous system pain, such as pain due to spinal cord or brain stem damage; lower back pain; sciatica; phantom limb pain, headache, including migraine and other vascular headaches, acute or chronic tension headache, cluster headache, temperomandibular pain and maxillary sinus pain; pain resulting from ankylosing spondylitis and gout; pain caused by increased bladder contractions; post operative pain; scar pain; and chronic non-neuropathic pain such as pain associated with fibromyalgia, HIV, rheumatoid and osteoarthritis, anthralgia and myalgia, sprains, strains and trauma such as broken bones; and post surgical pain in a mammal, including a human, comprising administering to said mammal an amount of a compound of the formula I or Group A compound, as defined above, or a pharmaceutically acceptable salt thereof, that is effective in treating such disorder or condition.

The present invention also relates to a method of treating a disorder or condition selected from the group consisting of pain resulting from soft tissue and peripheral damage, such as acute trauma; postherpetic neuralgia, trigeminal neuralgia, segmental or intercostal neuralgia and other neuralgias; pain associated with osteoarthritis and rheumatoid arthritis; musculo-skeletal pain, such as pain experienced after trauma; spinal pain, dental pain, myofascial pain syndromes, episiotomy pain, and pain resulting from burns; deep and visceral pain, such as heart pain, muscle pain, eye pain, orofacial pain, for example, odontalgia, abdominal pain, gynaecological pain, for example, dysmenorrhoea, labour pain and pain associated with endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, neuropathic lower back pain, HIV related neuropathic pain, diabetic neuropathic pain, and arachnoiditis; neuropathic and non-neuropathic pain associated with carcinoma, often referred to as cancer pain; central nervous system pain, such as pain due to spinal cord or brain stem damage; lower back pain; sciatica; phantom limb pain, headache, including migraine and other vascular headaches, acute or chronic tension headache, cluster headache, temperomandibular pain and maxillary sinus pain; pain resulting from ankylosing spondylitis and gout; pain caused by increased bladder contractions; post operative pain; scar pain; and chronic non-neuropathic pain such as pain associated with fibromyalgia, HIV, rheumatoid and osteoarthritis, anthralgia and myalgia, sprains, strains and trauma such as broken bones; and post surgical pain in a mammal, including a human, comprising administering to said mammal an amount of a compound of the formula I or Group A compound, as defined above, or a pharmaceutically acceptable salt thereof, that is effective in antagonizing the effect of substance P at its receptor site.

Other more specific methods of this invention include the above methods wherein the disorder or condition that is being treated is neuropathic pain.

Other more specific methods of this invention include the above methods wherein the disorder or condition that is being treated is HIV related neuralgia.

Other more specific methods of this invention include the above methods wherein the disorder or condition that is being treated is pain associated with fibromyalgia.

Other more specific methods of this invention include the above methods wherein the disorder or condition that is being treated is selected from neuropathic lower back pain, HIV related neuropathic pain, diabetic neuropathic pain, arachnoiditis and neuropathic and non-neuropathic pain associated with carcinoma.

Preferred methods of this invention include the above methods wherein the compound of the formula I that is employed in such method is one wherein R¹, R⁴, R⁵ and R⁷ are phenyl, R² is hydrogen, R³ is phenyl optionally substituted with chlorine, fluorine, (C₁-C₆) alkyl optionally substituted with from one to three fluorine atoms or (C₁-C₆) alkoxy optionally substituted with from one to three fluorine atoms, m is 0 and n is 3 or 4.

More specific preferred methods of this invention include the above methods wherein the compound of the formula I that is employed in such method is:
(2S,3S)-3-(5-tert-butyl-2-methoxybenzyl)amino-2-(3-trifluoromethoxyphenyl)piperidine;
(2S,3S)-3-(2-isopropoxy-5-trifluoromethoxybenzyl)amino-2-phenyl-piperidine;
(2S,3S)-3-(2-ethoxy-5-trifluoromethoxybenzyl)amino-2-phenyl-piperidine;
(2S,3S)-3-(2-methoxy-5-trifluoromethoxybenzyl)-amino-2-phenylpiperidine;
(2S,3S)-3(-5-tert-butyl-2-trifluoromethoxybenzyl)amino-2-phenylpiperidine;
2-(diphenylmethyl)-N-(2-methoxy-5-trifluoromethoxy-phenyl)methyl-1-azabicyclo[2.2.2]octan-3-amine;
(2S,3S)-3-[5-chloro-2-(2,2,2-trifluoroethoxy)-benzyl]amino-2-phenylpiperidine;
(2S,3S)-3-(5-tert-butyl-2-trifluoromethoxybenzyl)amino-2-phenylpiperidine;
(2S,3S)-3-(2-isopropoxy-5-trifluoromethoxybenzyl)amino-2-phenylpiperidine;
(2S,3S)-3-(2-difluoromethoxy-5-trifluoromethoxybenzyl)-amino-2-phenylpiperidine;
(2S,3S)-2-phenyl-3-[2-(2,2,2-trifluoroethoxybenzyl)-aminopiperidine; or
(2S,3S)-2-phenyl-3-(2-trifluoromethoxybenzyl)]aminopiperidine;
or a pharmaceutically acceptable salt thereof.

Other more specific embodiments of the present invention relate to the above methods wherein the compound of the formula I that is employed is selected from:
3-[N-(2-methoxy-5-trifluoromethoxybenzyl)-amino]-5,5-dimethyl-2-phenylpyrrolidine;
3-[N-(2-methoxy-5-trifluoromethoxy-benzyl)amino]-4,5-dimethyl-2-phenylpyrrolidine;
3-(2-cyclopropyloxy-5-trifluoromethoxybenzyl)amino-2-phenylpiperidine;
3-(2-cyclopropylmethoxy-5-trifluoromethoxybenzyl)amino-2-phenylpiperidine;
3-(2-difluoromethoxy-5-phenylbenzyl)amino-2-phenylpiperidine;
3-(5-cyclopropylmethoxy-2-difluoromethoxybenzyl)amino-2-phenylpiperidine;
3-(2-methoxybenzyl)amino-2-(3-trifluoromethoxyphenyl)-piperidine;
3-(2-methoxy-5-trifluoromethoxybenzyl)amino-2-(3-tri-fluoromethoxyphenyl)piperidine;
2-phenyl-3-(5-n-propyl-2-trifluoromethoxybenzyl)amino-piperidine;
3-(5-isopropyl-2-trifluoromethoxybenzyl)amino-2-phenylpiperidine;
3-(5-ethyl-2-trifluoromethoxybenzyl)amino-2-phenyl-piperidine;
3-(5-sec-butyl-2-trifluoromethoxybenzyl)amino-2-phenyl-piperidine;
3-(5-difluoromethoxy-2-methoxybenzyl)amino-2-phenyl-piperidine;
3-(2-methoxy-5-trifluoromethoxybenzyl)amino-2-phenylpyrrolidine;
3-(2-methoxy-5-trifluoromethoxybenzyl)amino-2-phenylhomopiperidine;
2-benzhydryl-3-(2-methoxy-5-trifluoromethoxy-benzyl)aminopyrrolidine;
2-benzhydryl-3-(2-methoxy-5-trifluoromethoxy-benzyl)aminohomopiperidine;
3-[2,5-bis-(2,2,2-trifluoroethoxy)benzyl]amino-2-phenylpiperidine;
2-phenyl-3-(3-trifluoromethoxybenzyl)aminopiperidine;
2-benzhydryl-3-(2-methoxy-5-trifluoromethoxybenzyl)-aminopiperidine;
1-(5,6-difluorohexyl)-3-(2-methoxy-5-trifluoromethoxy-benzyl)amino-2-phenylpiperidine;
1-(6-hydroxyhexyl)-3-(2-methoxy-5-trifluoromethoxy-benzyl)amino-2-phenylpiperidine;
3-phenyl-4-(2-methoxy-5-trifluoromethoxybenzyl)amino-2-azabicyclo[3.3.0]octane;
4-benzhydryl-5-(2-methoxy-5-trifluoromethoxybenzyl)-amino-3-azabicyclo[4.1.0]heptane;
4-(2-methoxy-5-trifluoromethoxybenzyl)amino-3-phenyl-2-azabicyclo[4.4.0]decane;
2-phenyl-3-(2-methoxy-5-trifluoromethoxybenzyl)-aminoquinuclidine;
8-benzhydryl-N-(2-methoxy-5-trifluoromethoxybenzyl)-9-azatricyclo[4.3.1.0^{4,9}]decan-7-amine;
9-benzhydryl-N-(2-methoxy-5-trifluoromethoxybenzyl)-10-azatricyclo[4.4.1.0^{5,10}]undecan-8-amine;
9-benzhydryl-N-(2-methoxy-5-trifluoromethoxybenzyl)-3-thia-10-azatricyclo-[4.4.1.0^{5,10}]undecan-8-amine;
8-benzhydryl-N-(2-methoxy-5-trifluoromethoxybenzyl)-9-azatricyclo[4.3.1.0^{4.9}]decan-7-amine;
5,6-pentamethylene-2-benzhydryl-3-(2-methoxy-5-trifluoromethoxybenzyl)amino-quinuclidine;
5,6-trimethylene-2-benzhydryl-3-(2-methoxy-5-trifluoromethoxybenzyl)amino-quinuclidine;
9-benzhydryl-N-((2-methoxy-5-trifluoromethoxyphenyl)-methyl)-3-oxa-10-azatricyclo-[4.4.1.0^{5,10}]undecan-3-amine;
8-benzhydryl-N-((2-methoxy-5-trifluoromethoxyphenyl)-methyl)-7-azatricyclo-[4.4.1.0^{5,10}]undecan-9-amine; and
2-benzhydryl-N-((2-methoxy-5-trifluoromethoxyphenyl)-methyl)-1-azabicyclo-[3.2.2]nonan-3-amine;
and the pharmaceutically acceptable salts of such compounds.

Other more specific embodiments of the present invention relate to the above methods wherein the compound of the formula I that is employed is one wherein Q is a group of the formula II wherein o is two or three and each of R¹ and R¹³ is phenyl or substituted phenyl.

Other more specific embodiments of the present invention relate to the above methods wherein the compound of the formula I that is employed is one wherein Q is a group of the formula III, R² is hydrogen and R³ is phenyl or substituted phenyl.

Other more specific embodiments of the present invention relate to the above methods wherein the compound of the formula I that is employed is one wherein Q is a group of the formula IV wherein I is one or two and each of R⁴ and R⁵ is phenyl or substituted phenyl.

Other more specific embodiments of the present invention relate to the above methods wherein the compound of the formula I that is employed is one wherein Q is a group of the formula V wherein n is zero or one and each of R⁴ and R⁵ is phenyl or substituted phenyl.

Other more specific embodiments of the present invention relate to the above methods wherein the compound of the formula I that is employed is one wherein Q is a group of the formula VI wherein p is one and each of R⁴ and R⁵ are phenyl or substituted phenyl.

Other more specific embodiments of the present invention relate to the above methods wherein the compound of the formula I that is employed is one wherein Q is a group of the formula VII wherein q is two, three or four, m is zero and R⁶ is phenyl or substituted phenyl.

Other more specific embodiments of the present invention relate to the above methods wherein a Group A compound is employed.

Other more specific embodiments of the present invention relate to the above methods wherein the Group A compound that is employed is selected from:
(2S,3S)-3-(6-methoxy-1-methyl-1-trifluoromethylisochroman-7-yl)methylamino-2-phenylpiperidine;
(2S,3S)-3-[(1R)-6-methoxy-1-methyl-1-trifluoromethylisochroman-7-yl]methylamino-2-phenylpiperidine;
(2S,3S)-N-(5-isopropyl-2-methoxyphenyl)methyl-2-di-phenylmethyl-1-azabicyclo[2.2.2]-octan-3-amine; and
(2S,3S)-N-(5-tert-butyl-2-methoxyphenyl)-methyl-2-diphenylmethyl-1-azabicyclo[2.2.2]-octan-3-amine;
and their pharmaceutically acceptable salts.

Other more specific methods of this invention include the above methods wherein the compound of formula I or Group A compound is administered to a human for the treatment of any two or more comorbid disorders or conditions selected from those disorders and conditions referred to in any of the above methods.

Other more specific methods of this invention include the above methods wherein the compound of formula I or Group A compound is administered to a human for the treatment of major depressive disorder and concomitant generalized anxiety disorder.

Other more specific methods of this invention include the above methods wherein the compound of formula I or Group A compound is administered to a human for the treatment of major depressive disorder and concomitant irritable bowel syndrome.

Other more specific methods of this invention include the above methods wherein the compound of formula I or Group A compound is administered to a human for the treatment of major depressive disorder and concomitant functional abdominal pain.

Other more specific methods of this invention include the above methods wherein the compound of formula I or Group A compound is administered to a human for the treatment of major depressive disorder and concomitant neuropathic pain.

Other more specific methods of this invention include the above methods wherein the compound of formula I or Group A compound is administered to a human for the treatment of major depressive disorder and concomitant premenstrual dysphoric disorder.

Other more specific methods of this invention include the above methods wherein the compound of formula I or Group A compound is administered to a human for the treatment of major depressive disorder and concomitant dysthymia.

Other more specific methods of this invention include the above methods wherein the compound of formula I or Group A compound is administered to a human for the treatment of major depressive disorder and concomitant fibromyalgia.

Other more specific methods of this invention include the above methods wherein the compound of formula I or Group A compound is administered to a human for the treatment of major depressive disorder and a concomitant somatoform disorder selected from somatization disorder, conversion disorder, body dysmorphic disorder, hypochondriasis, somatoform pain disorder and undifferentiated somatoform disorder.

Other more specific methods of this invention include the above methods wherein the compound of formula I or Group A compound is administered to a human for the treatment of generalized anxiety disorder and concomitant irritable bowel syndrome.

Other more specific methods of this invention include the above methods wherein the compound of formula I or Group A compound is administered to a human for the treatment of generalized anxiety disorder and concomitant functional abdominal pain.

Other more specific methods of this invention include the above methods wherein the compound of formula I or Group A compound is administered to a human for the treatment of generalized anxiety disorder and concomitant neuropathic pain.

Other more specific methods of this invention include the above methods wherein the compound of formula I or Group A compound is administered to a human for the treatment of generalized anxiety disorder and concomitant premenstrual dysphoric disorder.

Other more specific methods of this invention include the above methods wherein the compound of formula I or Group A compound is administered to a human for the treatment of generalized anxiety disorder and concomitant dysthymia.

Other more specific methods of this invention include the above methods wherein the compound of formula I or Group A compound is administered to a human for the treatment of generalized anxiety disorder and concomitant fibromyalgia.

Other more specific methods of this invention include the above methods wherein the compound of formula I or Group A compound is administered to a human for the treatment of generalized anxiety disorder and a concomitant somatoform disorder selected from somitization disorder, conversion disorder, hypochondriasis, somatoform pain disorder (or simply "pain disorder"), body dysmorphic disorder, undifferentiated somatoform disorder, and somatoform disorder not otherwise specified. See Diagnostic and Statistical manual of Mental Disortders, Fourth Edition (DSM-IV), American Psychiatric Association, Washington, D.C., May 1194, pp. 435-436.

Other more specific methods of this invention include the above methods wherein the compound of formula I or Group A compound is administered to a human for the treatment of major depressive disorder accompanied by one or more somatic symptoms selected from loss of appetite, sleep disturbances (*e*.*g*., insomnia, interrupted sleep, early morning awakening, tired awakening), loss of libido, restlessness, fatigue, constipation, dyspepsia, heart palpitations, aches and pains (*e*.*g*., headache, neck pain, back pain, limb pain, joint pain, abdominal pain), dizziness, nausea, heartburn, nervousness, tremors, burning and tingling sensations, morning stiffness, abdominal symptoms (e.g., abdominal pain, abdominal distention, gurgling, diarrhea), and the symptoms associated with generalized anxiety disorder (e.g., excessive anxiety and worry (apprehensive expectation), occurring more days than not for at least six months, about a number of events and activities, difficulty controlling the worry, etc.) See Diagnostic and Statistical manual of Mental Disorders, Fourth Edition (DSM-IV), American Psychiatric Association, Washington, D.C., May 1194, pp. 435-436 and 445-469. This document is incorporated herein by reference in its entirety.

Other more specific methods of this invention include the above methods wherein the formula I or Group A compound is administered to a human for the treatment of major depressive disorder accompanied by one or more somatic symptoms selected from fatigue, headache, neck pain, back pain, limb pain, joint pain, abdominal pain, abdominal distention, gurgling, diarrhea nervousness, and the symptoms associated with generalized anxiety disorder *(e.g.,* excessive anxiety and worry (apprehensive expectation), occurring more days than not for at least six months, about a number of events and activities, difficulty controlling the worry, etc. See Diagnostic and Statistical manual of Mental Disorders, Fourth Edition (DSM-IV), American Psychiatric Association, Washington, D.C., May 1194, pp. 435-436 and 445-469.

Other more specific methods of this invention include the above methods wherein the compound of formula I or Group A compound is administered to a human for the treatment of generalized anxiety disorder accompanied by one or more somatic symptoms selected from loss of appetite, sleep disturbances (e.g., insomnia, interrupted sleep, early morning awakening, tired awakening), loss of libido, restlessness, fatigue, constipation, dyspepsia, heart palpitations, aches and pains (e.g., headache, neck pain, back pain, limb pain, joint pain, abdominal pain), dizziness, nausea, heartburn, nervousness, tremors, burning and tingling sensations, morning stiffness, abdominal symptoms (*e*.*g*., abdominal pain, abdominal distention, gurgling, diarrhea), and the symptoms associated with major depressive disorder (*e*.*g*., sadness, tearfulness, loss of interest, ferafulness, helplessness, hopelessness, fatique,low self esteem, obsessive ruminations, suicidal thoughts, impaired memory and concentration, loss of motivation, paralysis of will, reduced appetite, increased appetite).

Other more specific methods of this invention include the above methods wherein the compound of formula I or Group A compound is administered to a human for the treatment of generalized anxiety disorder accompanied by one or more somatic symptoms selected from fatigue, headache, neck pain, back pain, limb pain, joint pain, abdominal pain, abdominal distention, gurgling, diarrhea nervousness, and the symptoms associated with major depressive disorder (e.g., sadness, tearfulness, loss of interest, fearfulness, helplessness, hopelessness, low self esteem, obsessive ruminations, suicidal thoughts, fatique, impaired memory and concentration, loss of motivation, paralysis of will, reduced apetite, increased appetite).

Compounds of formula I and the Group A compounds may contain chiral centers and therefore may exist in different enantiomeric and diastereomeric forms. This invention relates to all optical isomers and all stereoisomers of compounds of the formula I and the Group A compounds, both as racemic mixtures and as individual enantiomers and diastereoismers of such compounds, and mixtures thereof, and to all pharmaceutical compositions and methods of treatment defined above that contain or employ them, respectively. Individual isomers can be obtained by known methods, such as optical resolution, optically selective reaction, or chromatographic separation in the preparation of the final product or its intermediate.

The present invention also includes isotopically labelled compounds, which are identical to those recited in formula I or the Group A compounds, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula I and the Group A compounds of this invention and prodrugs thereof can generally be prepared by carrying out the procedures disclosed in the Schemes and/or in the Examples and Preparations below, by substituting a readily available isotopically labelled reagent for a non-isotopically labelled reagent.

### Detailed Description of the Invention

The compounds of the formula I may be prepared as described in the following reaction schemes and discussion. Unless otherwise indicated, R¹, R², R³, R⁴, R⁵, R⁶, R⁷, R⁸, R⁹, R¹⁰, R¹¹, R¹², R¹³, X, Z, Q, Y, m, n, o, p, q, x, y, and z in the reaction schemes and discussion that follow are defined as above.

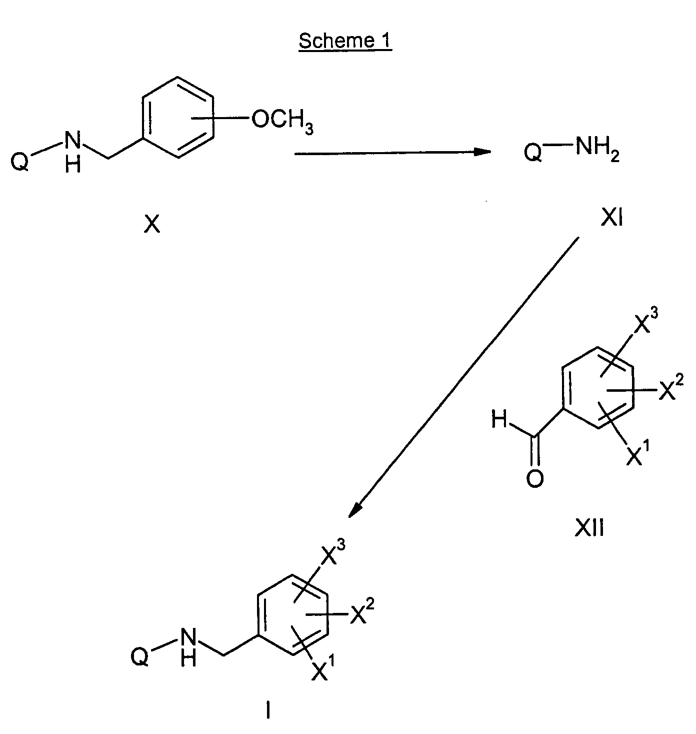

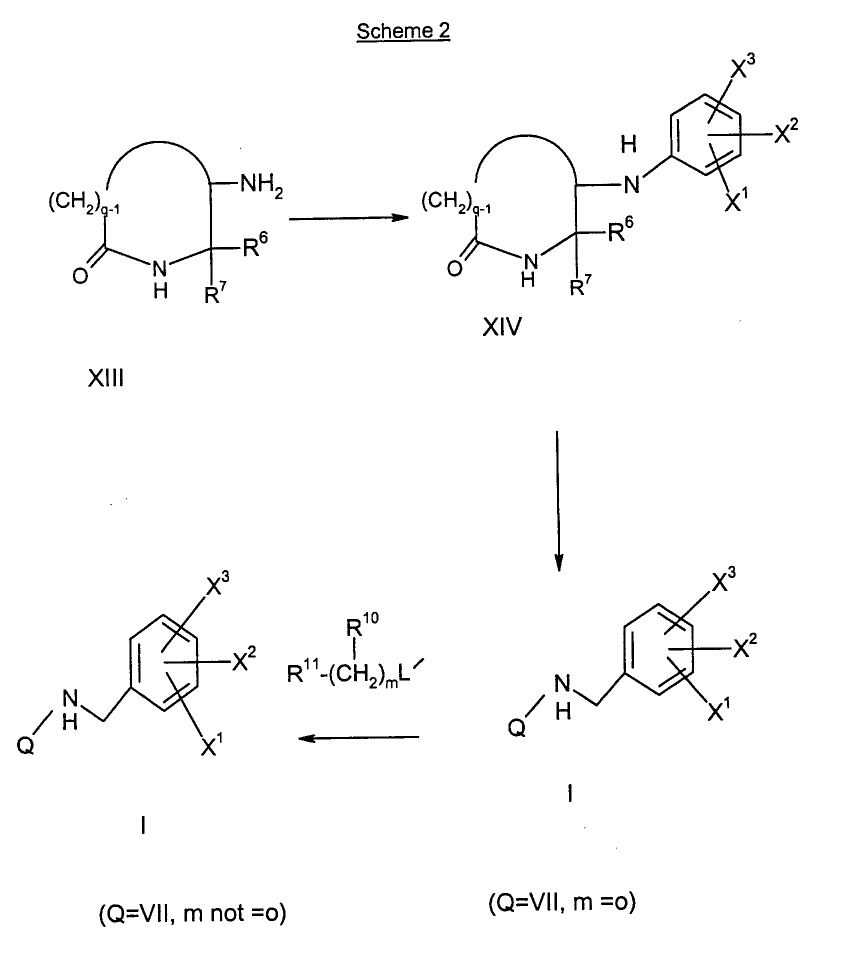

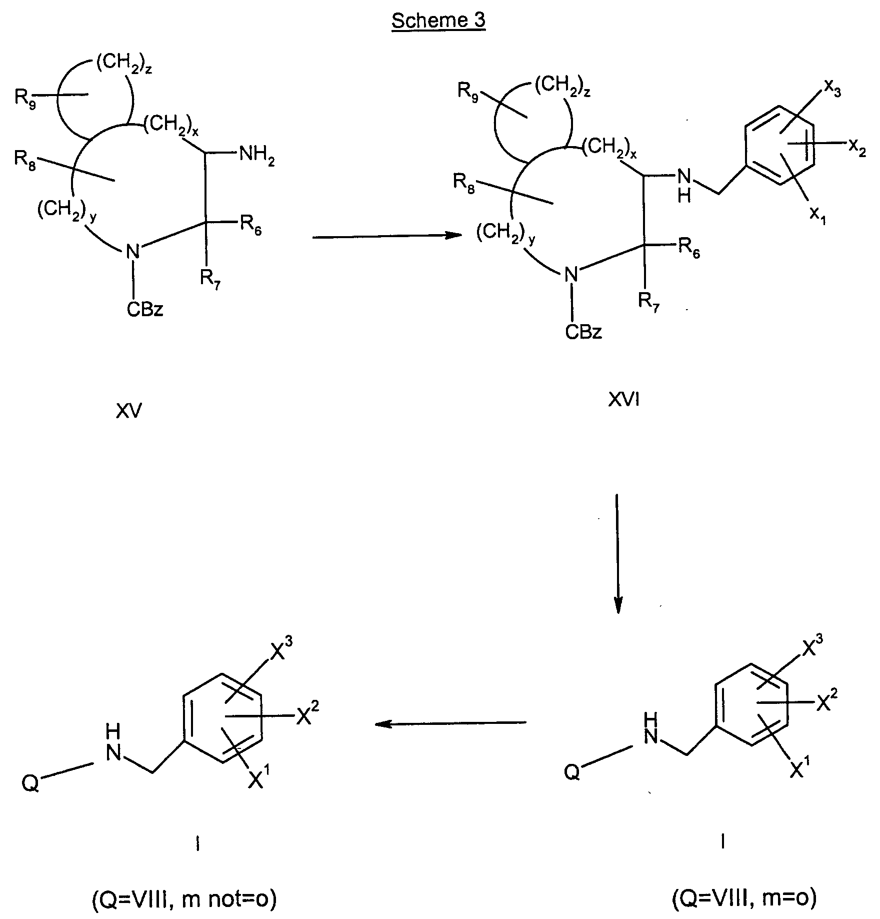

Compounds of the formula I may be prepared by the methods illustrated in schemes 1 and 2.

Referring to scheme 1, compounds of the formula X may be subjected to hydrolytic removal of the methoxybenzyl group using a strong mineral acid such as hydrochloric, hydrobromic or hydroiodic acid, at a temperature from about room temperature to about the reflux temperature of the acid. Preferably, the reaction is conducted in hydrobromic acid at the reflux temperature. This reaction, which yields the corresponding compounds of formula Xl, is usually carried out for a period of about 2 hours.

For those compounds of the formula X wherein Q is a group of the formula VII or VIII, it is preferable to remove the methoxybenzyl group by treating them with hydrogen in the presence of a metal containing catalyst such as platinum or palladium. Generally, this reaction is conducted in a reaction inert solvent such as acetic acid or a lower alcohol, at a temperature from about 0°C to about 50°C. (These compounds may also, alternatively, be treated with a dissolving metal such as lithium or sodium in ammonia at a temperature from about -30°C to about -78°C, or with a formate salt in the presence of palladium or with cyclohexane in the presence of palladium). Preferably, such compounds are treated with hydrogen in the presence of palladium on carbon in a mixture of methanol/ethanol in water or methanol/ethanol containing hydrochloric acid at a temperature of about 25°C.

The resulting compounds of the formula Xl may be converted to the corresponding compounds of the formula I by reaction with the appropriate compound of the formula XII (as depicted in scheme 1). This reaction is typically carried out in the presence of a reducing agent such as sodium cyanoborohydride, sodium triacetoxyborohydride, sodium borohydride, hydrogen and a metal catalyst, zinc and hydrochloric acid, borane dimethylsulfide or formic acid at a temperature from about -60°C to about 50°C. Suitable reaction inert solvents for this reaction include lower alcohols (*e*.*g*., methanol, ethanol and isopropanol), acetic acid and tetrahydrofuran (THF). Preferably, the solvent is acetic acid, the temperature is about 25°C, and the reducing agent is sodium triacetoxyborohydride.

Alternatively, the reaction of a compound of the formula Xl with a compound of the formula XII may be carried out in the presence of a drying agent or using an apparatus designed to remove azeotropically the water generated, to produce an imine of the formula 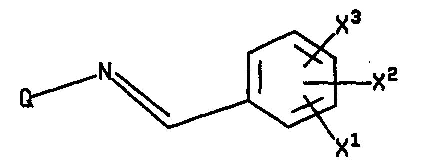 which is then reacted with a reducing agent as described above, preferably with sodium triacetoxyborohydride at about room temperature. The preparation of the imine is generally carried out in a reaction inert solvent such as benzene, xylene or toluene, preferably toluene, at a temperature from about 25°C to about 110°C, preferably at about the reflux temperature of the solvent. Suitable drying agents/solvent systems include titanium tetrachloride/dichloromethane, titanium isopropoxide/dichloromethane and molecular sieves/THF. Titanium tetrachloride/dichloromethane is preferred.

Compounds of the formula Xl may also be converted to the corresponding compounds of the formula I by reaction with the appropriate compound of the formula 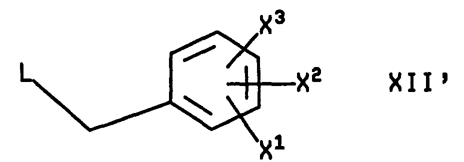 wherein L is a leaving group (*e*.*g*., chloro, bromo, iodo, tosylate or mesylate). This reaction is generally carried out in a reaction inert solvent such as dichloromethane or THF, preferably dichloromethane, at a temperature from about 0°C to about 60°C, preferably at about 25°C.

Compounds of the formula Xl may also be converted to the corresponding compounds of the formula I by reacting them with the appropriate compound of the formula 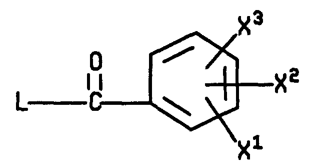 wherein L is defined as above or is imidazole, and then reducing the resulting amide. This reaction is typically carried out in an inert solvent such as THF or dichloromethane at a temperature from about -20°C to about 60°C, preferably in dichloromethane at about 0°C. Reduction of the resulting amide is accomplished by treatment with a reducing agent such as borane dimethylsulfide complex, lithium aluminum hydride or diisobutylaluminum hydride in an inert solvent such as ethyl ether or THF. The reaction temperature may range from about 0°C to about the reflux temperature of the solvent. Preferably, the reduction is accomplished using borane dimethylsulfide complex in THF at about 60°C.

When Q is a group of the formula II, the starting materials of the formula X may be prepared as described in United States Patent 5,162,339, which issued on November 10, 1992. This patent is incorporated herein by reference in its entirety.

When Q is a group of the formula III, the starting materials of the formula X may be prepared as described in United States Patent 5,451,586, which issued on September 19, 1995. This patent is incorporated herein by reference in its entirety.

When Q is a group of the formula IV, V or VI, the starting materials of the formula X may be prepared as described in United States Patent 5,482,354, which issued on June 6, 1995, United States Patent 5,641,786 which issued on June 27, 1997, United States Patent 5,698,568, which issued on December 16, 1997, United States Patent 5,821,248, which issued on October 13, 1998, and United States Patent 5,854,256, which issued on December 29, 1998. All of the foregoing patents are incorporated herein by reference in their entireties.

When Q is a group of the formula VII, the starting materials of the formula X may be prepared as described in United States Patent 5,232,929, which issued on August 3, 1993. This patent is incorporated herein by reference in its entirety.

When Q is a group of the formula VIII, the starting materials of the formula X may be prepared as described in United States Patent Application Serial No. 590,423, filed September 28, 1990 and World Patent Application WO 92/06079, which designates the United States and which was published on April 16, 1992. Both these applications are incorporated herein by reference in their entireties.

Scheme 2 illustrates an alternate method of preparing compounds of the formula I wherein Q is a group of the formula VII.

As shown in Scheme 2, reductive amination of a compound of the formula XII with sodium cyanoborohydride or sodium triacetoxyborohydride and a compound of the formula XIII yields a compound of the formula XIV. This reaction is typically carried out in a polar solvent such as acetic acid or a lower alkanol, at a temperature from about 0°C to about 50°C. Methanol is the preferred solvent and about 25°C is the preferred temperature. It is also preferable that the pH of the reaction mixture be from about 4 to about 5.

Reduction of the compound of formula XIV yields a compound of the formula I wherein Q is a group of the formula VII and m is zero. Suitable reducing agents include borane dimethylsulfide in THF, lithium aluminum hydride, borane in THF and sodium borohydride-titanium (IV) chloride. Best results are obtained by using borane dimethylsulfide in THF. The reaction may be carried out at temperatures from about room temperature to about 150°C, and is preferably carried out at the reflux temperature of the solvent.

The compounds of formula I so formed may be converted to a compound of the formula I wherein Q is a group of the formula VII and m is other than zero having the same stereochemistry by reacting them with the appropriate compound of the formula R¹⁰-(CH₂)ₘ-L', wherein L' is halo, mesylate or tosylate and wherein one of the carbon-carbon single bonds of said (CH₂)ₘ may optionally be replaced by a carbon-carbon double bond or a carbon-carbon triple bond, and wherein one of the carbons of said (CH₂)ₘ may optionally be substituted with R¹¹. This reaction is typically carried out in the presence of a base such as triethylamine or potassium t-butoxide, in a polar solvent such as methylene chloride or dichloroethane, and at a temperature from about room temperature to about 150°C. Preferably, the reaction is carried out at the reflux temperature in methylene chloride in the presence of triethylamine.

The starting materials of the formula XIII may be prepared as described in United States Patent 5,332,817, referred to and incorporated herein by reference above.

Scheme 3 illustrates an alternate method of making compounds of the formula I wherein Q is a group of the formula VIII.

As shown in scheme 3, reductive amination of a compound of the formula XII in the presence of a compound of the formula XV yields a compound of the formula XVI. Examples of reducing agents that may be used are hydrogen in the presence of a metal catalyst, sodium borohydride, sodium cyanoborohydride and sodium triacetoxyborohydride. This reaction is generally carried out in a polar solvent such as acetic acid or a lower alkanol, in the presence of a dehydrating agent such as molecular sieves, at a temperature from about 0 to about 50°C. Methanol is the preferred solvent and 25°C is the preferred temperature. It is also preferable that the pH of the reaction mixture be from about 4 to about 5.

Alternatively, compounds of the formula XVI may be formed by acylating a compound of the formula XV with a compound having the formula 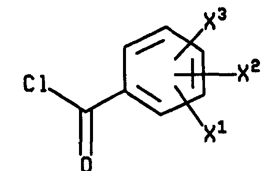 and then reducing the resulting amide. The acylation is generally conducted in a polar solvent (*e*.*g*., dichloromethane, THF or ethyl ether), at a temperature from about 0 to about 60°C. The preferred solvent is dichloromethane and the preferred temperature is about 25°C. Examples of reducing agents that may be used to reduce the amide are lithium aluminum hydride and borane dimethyl sulfide. The reduction is typically carried out in a polar solvent (e.g., ether, THF or DME) at a temperature from about 0°C to about the reflux temperature of the solvent, preferably at about room temperature.

The compounds of formula XVI may be converted into the corresponding compounds of formula I wherein Q is a group of the formula VIII and m is zero by reacting them with ammonium formate in the presence of palladium on charcoal (*e*.*g*., 10% palladium on charcoal). Usually, a polar solvent such as ethyl acetate or a lower alkanol is used, and the reaction is run at a temperature from about room temperature to about 150°C for about 0.5 to about 24 hours. Preferably, the reaction is conducted in ethanol at room temperature for about 3 to about 24 hours.

The compounds of the formula I prepared by the foregoing procedure may be converted into compounds that are identical but for the fact that m is not equal to zero using the procedure described above for preparing compounds of the formula I wherein Q is a group of the formula VII and m is not equal to zero.

The starting materials of the formula XV may be prepared as described in United States Patent application Serial No. 590,423, filed September 28, 1990, and World Patent Application WO 92/06079, which designates the United States and which was published on April 16, 1992. These applications are incorporated herein by reference in their entireties.

The preparation of other compounds of the formula I not specifically described in the foregoing experimental section can be accomplished using combinations of the reactions described above that will be apparent to those skilled in the art.

In each of the reactions discussed or illustrated in schemes 1 to 3 above, pressure is not critical unless otherwise indicated. Pressures from about 0.5 atmospheres to about 5 atmospheres are generally acceptable, and ambient pressure, i.e., about 1 atmosphere, is preferred as a matter of convenience.

The Group A compounds may be prepared as described in European Patent Application 1,032,571, which was published on September 6, 2000, and in United States Patent 5,807,867, which issued on September 15, 1998. These publications are incorporated herein by reference in their entireties.

The compounds of the formula I and the Group A compounds that are employed in the novel methods of this invention and the pharmaceutically acceptable salts thereof are useful as substance P antagonists, *i*.*e*., they possess the ability to antagonize the effects of substance P at its receptor site in mammals, and therefore they are able to function as therapeutic agents in the treatment of the aforementioned disorders and diseases in an afflicted mammal.

The compounds of the formula I and the Group A compounds that are basic in nature are capable of forming a wide variety of different salts with various inorganic and organic acids. Although such salts must be pharmaceutically acceptable for administration to animals, it is often desirable in practice to initially isolate a compound of the formula I or a Group A compound from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained.

Those compounds of the formula I and the Group A compounds that are also acidic in nature, *e*.*g*., where R¹ of formula I is carboxyphenyl, are capable of forming base salts with various pharmacologically acceptable cations. Examples of such salts include the alkali metal or alkaline-earth metal salts and particularly, the sodium and potassium salts. These salts are all prepared by conventional techniques. The chemical bases which are used as reagents to prepare the pharmaceutically acceptable base salts of this invention are those which form non-toxic base salts with the acidic compounds of formula I and the Group A compounds. Such non-toxic base salts include those derived from such pharmacologically acceptable cations as sodium, potassium calcium and magnesium, etc. These salts can easily be prepared by treating the corresponding acidic compounds with an aqueous solution containing the desired pharmacologically acceptable cations, and then evaporating the resulting solution to dryness, preferably under reduced pressure. Alternatively, they may also be prepared by mixing lower alkanolic solutions of the acidic compounds and the desired alkali metal alkoxide together, and then evaporating the resulting solution to dryness in the same manner as before. In either case, stoichiometric quantities of reagents are preferably employed in order to ensure completeness of reaction and maximum yields of the desired final product.

The compounds of formula I and the Group A compounds and their pharmaceutically acceptable salts exhibit substance P receptor-binding activity and therefore are of value in the treatment of a wide variety of clinical conditions the treatment or prevention of which are effected or facilitated by a decrease in substance P mediated neurotransmission. Such conditions include sleep disorders (e.g., sleep apnea, insomnia, somnambulism, sleep deprivation, REM sleep disorders, hypersomnia, parasomnias, sleep-wake cycle disorders, narcolepsy, sleep disorders associated with shift work or irregular work schedules, and other sleep disorders); autism; pervasive development disorder; rheumatoid arthritis; osteoarthritis; fibromyalgia; human immunodeficiency virus (HIV) infections; dissociative disorders such as body dysmorphic disorders; eating disorder such as anorexia and bulimia; ulcerative colitis; Crohn's disease; chronic fatigue syndrome; sudden infant death syndrome (SIDS); overactive bladder; chronic cystitis; chemotherapy induced cystitis; cough, angiotensin converting enzyme (ACE) induced cough; itch; hiccups; premenstrual syndrome: premenstrual dysphoric disorder; amenorrheic disorders such as desmenorrhea; obesity; epilepsy: movement disorders such as primary movement disorders, spasticities, Scott's syndrome, Tourette's syndrome, palsys (*e*.*g*., Bell's palsy, cerebral palsy, birth palsy, brachial palsy, wasting palsy, ischemic palsy, progressive bulbar palsy and other palsys), amyolateral sclerosis (ALS), akinetic-rigid disorders, akinesias, dyskinesias (e.g., familial paroxysmal dyskinesia, tardive dyskinesia, tremor, chorea, myoclonus, tics and other dyskinesias) restless leg syndrome and movement disorders associated with Parkinson's disease or Huntington's disease; mastalgia syndromes; motion sickness; immune dysfunctions (e.g., stress induced immune dysfunctions such as idiopathic immune dysfunctions, post infection immune dysfunctions, post lumpectomy immune dysfunctions, porcine stress syndrome, bovine shipping fever, equine paroxysmal fibrillation, confinement dysfunction in chicken, sheering stress in sheep, and human-animal interaction stress in dogs); generalized anxiety disorder; panic disorder; phobias, including social phobia, agoraphobia, and specific phobias; obsessive-compulsive disorder; post-traumatic stress disorder; depression including major depression, single episode depression, recurrent depression, child abuse induced depression, postpartum depression and dysthemia; cyclothymia; bipolar disorder; neurocardiac disorders such as neurocardiac syncope, neurogenic syncope, hypersensitive Carotid sinus, neurovascular syndrome and arrythmias including arrythmias secondary to gastrointestinal disturbances; addiction disorders involving addictions to behaviors (e.g., addictions to gambling and other addictive behaviors); AIDS related neuralgias; epilepsy; and attention deficit hyperactivity. Hence, these compounds are readily adapted to therapeutic use as substance P antagonists for the treatment of any of the foregoing clinical conditions in mammals, including humans.

The compounds of the formula I and the Group A compounds and the pharmaceutically acceptable salts thereof can be administered via either the oral, parenteral or topical routes. In general, these compounds are most desirably administered in dosages ranging from about 5.0 mg up to about 1500 mg per day, although variations will necessarily occur depending upon the weight and condition of the subject being treated and the particular route of administration chosen. However, a dosage level that is in the range of about 0.07 mg to about 21 mg per kg of body weight per day is most desirably employed. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The compounds of formula I and the Group A compounds and their pharmaceutical acceptable salts, when used in the novel methods of this invention, can be administered alone or in combination with pharmaceutically acceptable carriers or diluents by either of the three routes previously indicated, and such administration may be carried out in single or multiple doses. More particularly, the novel therapeutic agents of this invention can be administered in a wide variety of different dosage forms, *i*.*e*., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the compounds of the formula I and their pharmaceutically acceptable salts are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the formula I or a Group A compound or a pharmaceutically acceptable salt thereof in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

Additionally, it is also possible to administer the compounds of the formula I and the Group A compounds and their pharmaceutically acceptable salts topically and this may preferably be done by way of creams, jellies, gels, pastes, ointments and the like, in accordance with standard pharmaceutical practice.

The activity of the compounds of the formula I and the Group A compounds and their pharmaceutically acceptable salts as substance P antagonists may be determined by their ability to inhibit the binding of substance P at its receptor sites in bovine caudate tissue, employing radioactive ligands to visualize the tachykinin receptors by means of autoradiography. The substance P antagonizing activity of the herein described compounds may be evaluated by using the standard assay procedure described by M. A. Cascieri *et al*., as reported in the Journal of Biological Chemistry, Vol. 258, p. 5158 (1983). This method essentially involves determining the concentration of the individual compound required to reduce by 50% the amount of radiolabelled substance P ligands at their receptor sites in said isolated cow tissues, thereby affording characteristic IC₅₀ values for each compound tested.

In this procedure, bovine caudate tissue is removed from a -70°C freezer and homogenized in 50 volumes (w./v.) of an ice-cold 50 mM Tris (i.e., trimethamine which is 2-amino-2-hydroxymethyl-1,3-propanediol) hydrochloride buffer having a pH of 7.7. The homogenate is centrifuged at 30,000 x G for a period of 20 minutes. The pellet is resuspended in 50 volumes of Tris buffer, rehomogenized and then recentrifuged at 30,000 x G for another twenty- minute period. The pellet is then resuspended in 40 volumes of ice-cold 50 mM Tris buffer (pH 7.7) containing 2 mM of calcium chloride, 2 mM of magnesium chloride, 40 g/ml of bacitracin, 4 g/ml of leupeptin, 2 g of chymostatin and 200 g/ml of bovine serum albumin. This step completes the production of the tissue preparation.

The radioligand binding procedure is then carried out in the following manner, viz., by initiating the reaction via the addition of 100 l of the test compound made up to a concentration of 1 M, followed by the addition of 100 l of radioactive ligand made up to a final concentration 0.5 mM and then finally by the addition of 800 l of the tissue preparation produced as described above. The final volume is thus 1.0 ml, and the reaction mixture is next vortexed and incubated at room temperature (ca. 20°C) for a period of 20 minutes. The tubes are then filtered using a cell harvester, and the glass fiber filters (Whatman GF/B) are washed four times with 50 mM of Tris buffer (pH 7.7), with the filters having previously been presoaked for a period of two hours prior to the filtering procedure. Radioactivity is then determined in a Beta counter at 53% counting efficiency, and the IC₅₀ values are calculated by using standard statistical methods.

The present invention is illustrated by the following examples. It will be understood, however, that the invention is not limited to the specific details of these examples.

### EXAMPLE 1

### 2-(Diphenylmethyl)-N-((2-difluoromethoxy)phenyl)methyl-1-azabicyclo[2.2.2]octan-3-amine

### A. 2-(Difluoromethoxy)benzaldehyde:

To a 500 mL three-necked round-bottomed flask equipped with condenser and gas inlet tube were added 5.0 g (40.98 mmol) salicylaldehyde, 150 mL dioxane, and 150 mL (164 mmol) of a 1.1 N aqueous solution of sodium hydroxide. Chlorodifluoromethane gas was bubbled through the reaction mixture as it was heated to 60°C, and the reaction mixture was stirred at this temperature for 2 hours. The reaction mixture was then cooled and extracted with ether. The organic layer was dried over sodium sulfate, filtered and evaporated. The residue was chromatographed on silica gel using hexane/ethyl acetate as eluant to afford a light yellow oil, 1.63 g (23%).

¹H NMR ( , CDCl₃): 6.64 (t, J=72.7 (H-F), 1H), 7.16 (d, J=7, 1H), 7.24 (t, J=7, 1H), 7.53 (m, 1H), 7.81 (m, 1H), 10.29 (s, 1H).

¹³C-NMR (CDCl₃): 112.2, 115.6, 115.645, 115.7, 119.1, 119.2, 119.5, 125.6, 125.7, 125.8, 125.9, 127.5, 128.8, 128.9, 135.7, 152.71, 152.73, 188.4.

IR (cm⁻¹, neat): 1700 (C=O).

MS (%): 172 (100, parent), 171 (48), 122 (45), 121 (82), 120 (69), 104 (37), 95 (40), 92 (55), 91 (49), 76 (39), 65 (49), 63 (76), 51 (81).

Anal. Calc'd for C₈H₆F₂O₂•1/4H₂O: C 54.50, H 3.71. Found: C 54.68, H 3.33.

### B. 2-(Diphenylmethyl)-N-((2-difluoromethoxy)-phenyl)methyl-1-azabicyclo[2.2.2]octan-3-amine

To a 25 mL round-bottomed flask equipped with a nitrogen inlet were added 500 mg (1.71 mmol) 2-diphenylmethyl-1-azabicyclo[2.2.2]octan-3-amine (prepared according to the method of Warawa, et al., J. Med. Chem., 17, 497 (1974)), 8.5 mL methanol, 383 mg (2.23 mmol) 2-(difluoromethoxy)-benzaldehyde, and 216 mg (3.42 mmol) sodium cyanoborohydride. The reaction was stirred at room temperature for 30 hours, partitioned between ethyl acetate and water. The organic layer was separated, washed with brine, dried over sodium sulfate, and evaporated. To remove the last traces of unreacted amine, the mixture was treated with sodium triacetoxyborohydride in acetic acid at room temperature for 16 hours, then worked up with aqueous sodium hydroxide and methylene chloride. The residue was crystallized from isopropanol to afford a white solid, m.p. 144-147°C, 206 mg (27%).

¹H NMR ( , CDCl₃): 1.27 (m, 1H), 1.4-1.8 (m, 2H), 1.90 (m, 1H), 2.05 (m, 1H), 2.63 (m, 1H), 2.78 (m, 2H), 2.88 (m, 1H), 3.19 (m, 1H), 3.45 (AB_{q}, J_{AB}=13.5, v=105.5, 2H), 3.72 (dd, J=8, 12, 1H), 4.43 (d, J=12, 1H), 6.31 (t, J=74 (H-F), 1H), 6.55 and 7.0-7.4 (m, 14H).

¹³C-NMR (CDCl₃): 20.0, 24.9, 25.4, 42.0, 45.8, 49.4, 49.5, 55.0, 61.8, 116.3, 119.0, 125.4, 126.0, 126.5, 127.5, 127.8, 127.9, 128.0, 128.4, 128.5, 128.6, 129.1, 129.2, 130.0, 131.6, 143.2, 145.2, 149.3.

IR (cm⁻¹, neat): 2940 (C-H), 1599 (C=C).

MS (%): 449 (<1, parent+1), 291 (51), 281 (100), 84 (66), 49 (69).

Anal. Calc'd for C₂₈H₃₀F₂N₂O: C 74.98, H 6.74, N 6.25. Found: C 74.72, H 6.70, N 6.23.

### EXAMPLE 2

### (2S,3S)-N-(2-Methoxy-5-trifluoromethoxyphenyl)methyl-2-diphenylmethyl-1-azabicyclo[2,2,2]octane-3-amine methanesulfonic acid salt

The title compound was prepared in a manner similar to the procedure described in Example 1, by replacing 2-(difluoromethoxy)benzaldehyde with 2-methoxy-5-trifluoromethoxybenzaldehyde in Step B.

M.p. 135°C.

¹H NMR (CDCl₃) 1.8-2.3 (m, 2H), 2.2-2.8 (m, 6H), 2.66 (s, 6H), 3.56 (s, 3H), 3.3-3.7 (m, 3H), 3.90 (m, 3H), 4.16 (m, 2H), 5.06 (m, 1H), 5.20 (br, 1H), 5.50 (m, 1H), 5.60 (br, 1H), 6.77 (d, 1H, J=9.2), 7.02 (m, 1H), 7.2-7.8 (m, 11H), 8.00 (br, 1H), 10.8 (br, 1H).

IR (cm⁻¹, KBr): 3180, 3140, 3000, 1500, 1200, 1062, 782.

### EXAMPLE 3

### (2S,3S)-2-Phenyl-3-[2-(2,2,2-trifluoroethoxy)benzyl]-aminopiperidine hydrochloride

### A. 2-(2.2.2-Trfluoroethoxy)benzaldehyde

Under a nitrogen atmosphere in a round-bottom flask equipped with a reflux condenser were placed 0.2 g (1 mmol) of 2-(2,2,2-trifluoroethoxy)benzonitrile (J. Org. Chem., 377 (1983)) and 5 mL of formic acid. To this solution was added ca. 0.2 g of Raney nickel, and the mixture was heated at reflux for 90 minutes. The mixture was filtered through diatomaceous earth, and the filter cake was rinsed with water and chloroform (CHCl₃). The layers were separated, and the aqueous phase was extracted with three portions of chloroform. The combined organic fractions were washed with saturated aqueous sodium bicarbonate and water, dried over sodium sulfate (Na₂SO₄) and concentrated (rotary evaporator) to obtain 176 mg of the title compound as a yellow solid, m.p. 33-34°C.

### B. (2S,3S)-2-Phenyl-3-[2-(2,2,2-trifluoroethoxy)-benzyl]aminopiperidine hydrochloride

Under a nitrogen atmosphere in a round-bottom flask were placed 112 mg (0.63 mmol) of (2S, 3S)-3-amino-2-phenylpiperidine, 155 mg (0.76 mmol) of the aldehyde prepared in step A above and ca. 2 mL of acetic acid, and the solution was stirred at room temperature for 1 hour. To the system were added 294 mg (1.39 mmol) of sodium triacetoxyborohydride in portions, and the mixture was stirred at room temperature overnight. The mixture was concentrated with a rotary evaporator and partitioned between 1M aqueous sodium hydroxide (NaOH) and methylene chloride (CH₂Cl₂). The layers were separated, and the aqueous phase was extracted with three portions of CH₂Cl₂. The combined organic fractions were extracted with three portions of 2N aqueous HCI, the extracts were made basic with 2N aqueous NaOH, and the mixture was extracted with four portions of CH₂Cl₂. These CH₂Cl₂ extracts were dried (Na₂SO₄) and concentrated. The resulting oil was dissolved in ca. 2 mL ethyl acetate and treated with ether saturated with hydrogen chloride (HCI). The resulting white solid (73 mg, m.p. > 275°C) was collected. This material was converted to its free base by partitioning between 1N aqueous NaOH and CH₂Cl₂. The free base (58 mg) was purified by flash column chromatography eluting with chloroform (CHCl₃) followed by 1:19 methanol/CHCl₃ to obtain 32 mg of oil. Conversion of the free base to the corresponding hydrochloride salt as described above afforded 17 mg of the title compound, m.p. > 275°C.

¹H NMR (free base, CDCl₃) 1.44 (m, 1H), 1.63 (m, 1H), 1.88 (m, 1H), 2.1 (m, 1H), 2.80 (m, 2H), 3.26 (m, 1H), 3.38 (d, 1H, J=15), 3.66 (d, 1H, J=15), 3.88 (s, 1H), 4.08 (m, 2H), 6.68 (d, 1 H, J=6), 6.90 (m, 1 H), 6.98 (d, 1 H, J=6), 7.16 (m, 1H), 7.26 (m, 5H).

HRMS Calc'd for C₂₀H₂₄F₃N₂O₃ (parent + 1): 365.1835. Found: 365. 1980.

Anal. Calc'd for C₂₀H₂₃F₃N₂O•2HCl•1/3H₂O: C, 54.19, H, 5.84; N, 6.32. Found: C, 54.22, H, 5.57, N, 6.28.

### EXAMPLE 4

### (2S,3S)-3-(2-Methoxy-5-trifluoromethoxybenzyl)amino-2-phenylpiperidine hydrochloride salt

### A. 2-Methoxy-5-trifluoromethoxybenzaldehyde

Under a nitrogen atmosphere in a round-bottom flask were placed 3.63 mL (28 mmol) of 4-trifluoromethoxyphenol and 25 mL of acetone. To this stirring solution were added 7.75 g (56 mmol) of potassium carbonate and 3.48 mL (56 mmol) of methyl iodide, and the reaction mixture was stirred at room temperature overnight. The solids were removed by suction filtration and the filter cake was rinsed with acetone. The filtrate was concentrated to obtain 6.5 g of a solid/oil mixture. This mixture was diluted with CHCl₃ and filtered and the filtrate was concentrated to afford 5.5 g of 1-methoxy-4-trifluoromethoxybenzene as a yellow oil.

¹H NMR (CDCl₃) 3.78 (s, 3H), 6.83 (d, 1H, J=12), 7.10 (d, 1H, J=12). Mass spectrum m/z: 192 (parent).

Under a nitrogen atmosphere in a round-bottom flask were placed the 1-methoxy-4-trifluoromethoxybenzene (5.5 g, 29 mmol) and 110 mL of CH₂Cl₂. To the system, cooled in an ice/acetone bath, were added 3.77 mL (34 mmol) of titanium tetrachloride (TiCl₄) over a period of ca. 1 minute. The reaction mixture was stirred for 30 minutes and 5.69 mL (63 mmol) of ,-dichloromethylmethyl ether was added to the system. The ice bath was allowed to expire and the mixture was stirred at room temperature overnight. The mixture was poured carefully into water and extracted with three portions of CH₂Cl₂. These combined extracts were washed with water and brine, dried (Na₂SO₄) and concentrated to obtain 6.06 g of an oil. The crude material was purified by flash column chromatography (250 g of silica gel) using 1:9 ethyl acetate/hexanes as the eluant to obtain 920 mg of the title compound with a slight impurity and 3.27 g of pure title compound.

¹H NMR (CDCl₃) 3.94 (s, 3H), 7.00 (d, 1H, J=9), 7.38 (dd, 1H, J=3, 9), 7.66 (d, 1H, J=3), 10.4 (s, 1H). Mass spectrum m/z: 220 (parent).

### B. (2S,3S)-3-(2-methoxv-5-trifluoromethoxybenzyl)-amino-2-phenylpiperidine hydrochloride salt

Under a nitrogen atmosphere in a round-bottom flask were placed 525 mg (2.4 mmol) of 2-methoxy-5-trifluoromethoxybenzaldehyde, 350 mg (2.0 mmol) of (2S, 3S)-3-amino-2-phenylpiperidine and 5 mL of acetic acid. The reaction mixture was stirred at room temperature for 3 days and concentrated with a rotary evaporator. The residue was partitioned between 1N aqueous sodium hydroxide and chloroform (CHCl₃) and the mixture was extracted with three portions of chloroform. The combined chloroform extracts were extracted with three portions of 1N aqueous hydrochloric acid. The combined HCI extracts were made basic with concentrated aqueous sodium hydroxide and extracted with four portions of chloroform. The chloroform extracts were dried (Na₂SO₄) and concentrated with a rotary evaporator to obtain 760 mg of an oil. The oil was dissolved in ethyl acetate, and ether saturated with hydrogen chloride (HCI) was added to the solution. The resulting white solid was collected by suction filtration and washed with ether to obtain 600 mg of the title compound, m.p. > 250°C.

¹H NMR (free base, CDCl₃) 1.36 (s, 1H), 1.54 (m, 1H), 1.86 (m, 1H), 2.06 (m, 1H), 2.76 (m, 2H), 3.22 (m, 1H), 3.32 (d, 1H, J=15), 3.48 (s, 3H), 3.58 (d, 1H, J=15), 3.85 (d, 1H, J=3), 6.57 (d, 1H, J=9), 6.80 (d, 1H, J=3), 6.92 (dd, 1H, J=3, 9), 7.22 (m, 5H).

HRMS Calc'd for C₂₀H₂₃F₃N₂O₂: 380.1711. Found: 380.1704.

Anal. Calc'd for C₂₀H₂₃F₃N₂O₂•2HCl•0.2H₂O: C 52.57, H 5.60, N 6.13. Found: C 52.58, H 5.40, N 5.97.

### EXAMPLE 5

### (2S,3S)-1-(5,6-Dimethoxyhexyl)-3-(2-methoxv-5-tri-fluoromethoxybenzyl)amino-2-phenylpiperidine hydrochloride

Under a nitrogen atmosphere in a round-bottom flask were placed 250 mg (0.66 mmol) of (2S, 3S)-3-(2-methoxy-5-trifluoromethoxybenzyl)amino-2-phenylpiperidine, 2 mL of tetrahydrofuran (THF) and 0.28 mL (2.0 mmol) of triethylamine. To the system were added 475 mg (2.0 mmol) of 5,6-dimethoxy-1-methylsulfonyloxyhexane (prepared from 1,5,6-hexanetriol by sequential acetonide formation (acetone, p-toluenesulfonic acid), acetylation (acetyl chloride, triethylamine, THF), acetonide cleavage (60% acetic acid/water), dimethylation (sodium hydride, methyl iodide, THF), deacetylation (sodium methoxide, methanol) and methanesulfonate ester formation (methanesulfonyl chloride, triethylamine, THF)), and the mixture was heated at 50-60°C for four days. The reaction mixture was partitioned between CHCl₃ and saturated aqueous sodium bicarbonate and extracted with three portions of CHCl₃. The combined organic fractions were dried (Na₂SO₄), filtered and concentrated to obtain 853 mg of an orange oil. The crude material was purified by flash column chromatography (35 g of silica gel) using 1:19 methanol/chloroform as the eluant to obtain 185 mg of yellow oil. The oil was dissolved in ethyl acetate and ether saturated with HCI was added to the solution. The mixture was concentrated and the residue was triturated with ether to obtain 190 mg of the title compound.

¹H NMR (free base, CDCl₃) 1.15 (m, 2H), 1.38 (m, 6H), 1.76 (m, 2H), 1.96 (m, 3H), 2.50 (m, 2H), 3.16 (m, 2H), 3.26 (m, 9H), 3.46 (s, 3H), 3.58 (d, 1H, J=15), 6.52 (d, 1H, J=9), 6.69 (m, 1H), 6.86 (m, 1H), 7.22 (m, 5H).

HRMS calc'd for C₂₈H₃₉F₃N₂O₄: 524.28616. Found: 524.28634.

Anal. Calc'd for C₂₈H₃₉F₃N₂O₄•2HCl•0.75H₂O: C 55.03, H 7.00, N 4.58. Found: C 55.04, H 7.12, N 4.51.

### EXAMPLE 6

### (2S,3S)-2-Phenyl-3-(2-trifluoromethoxybenzyl)amino-piperidine hydrochloride salt

Under a nitrogen atmosphere in a round-bottom flask were placed 3.0 mL (23 mmol) of trifluoromethoxybenzene and 25 mL of benzene. The system was cooled in ice/acetone bath, and 4.1 mL (45 mmol) of , -dichloromethylmethyl ether was added to the stirring solution. To the system was added 6.13 g (46 mmol) of aluminum chloride (AlCl₃) in portions. After this addition was complete, the reaction mixture was allowed to warm gradually to room temperature and stirred at room temperature overnight. The reaction mixture was poured slowly into water and extracted with three portions of dichloromethane. -The combined organic fractions were washed with water, dried (Na₂SO₄) and concentrated with a rotary evaporator to obtain 3.7 g of oil. This material, containing a mixture of 4- and 2-trifluoromethoxybenzaldehyde, was subjected to flash column chromatography (160 g of silica gel) using 1:49 ethyl acetate/hexanes as the eluant to obtain 500 mg of material enriched in 2-trifluoromethoxy-benzaldehyde.

Under a nitrogen atmosphere in a round-bottom flask were placed 155 mg (0.88 mmol) of (2S,3S)-3-amino-2-phenylpiperidine, the aldehyde obtained above and 2 mL of acetic acid. To the system were added 370 mg (1.8 mmol) of sodium triacetoxyborohydride and the mixture was stirred at room temperature overnight. The mixture was concentrated and the residue was partitioned between 1N aqueous sodium hydroxide and dichloromethane and extracted with three portions of dichloromethane. The combined organic fractions were extracted with three portions of 1N HCI. The acid extracts were made basic with 1N aqueous NaOH and extracted with three portions of dichloromethane. The dichloromethane extracts were dried and concentrated to afford 190 mg of oil, which was subjected to flash column chromatography (5 g of silica gel) using 1:9 methanol/chloroform as the eluant to obtain 95 mg of the free base of the title compound. The free base was dissolved in ethyl acetate, and ether saturated with HCI was added to the solution. The resulting white solid was collected by suction filtration and rinsed with ether to obtain 72 mg of the title compound, m.p. 231-233°C.

¹H NMR (free base, CDCl₃) 1.40 (m, 1H), 1.60 (m, 1H), 1.84 (m, 1H), 2.05 (m, 1H), 2.78 (m, 2H), 3.22 (m, 1H), 3.42 (d, 1H, J=15), 3.56 (d, 1H, J=15), 3.86 (d, 1H, J=3), 7.08 (m, 4H), 7.24 (m, 5H). Mass spectrum: m/z 350 (parent).

Anal. Calc'd for C₁₉H₂₁F₃N₂O•2HCl•0.25H₂O: C 53.34, H 5.54, N 6.54. Found: C 53.19, H 5.40, N 6.54.

### EXAMPLE 7

### (2S,3S)-3-(2-Hydroxy-5-trifluoromethoxybenzyl)amino-2-phenylpiperidine Hydrochloride

### A. 2-Hydroxy-5-trifluoromethoxybenzaldehyde

Under a nitrogen atmosphere, in a round-bottom flask were placed 300 mg (1.4 mmol) of 2-methoxy-5-trifluoromethoxybenzaldehyde and 30 ml of dichloromethane. To the system, cooled in a dry ice acetone bath, were added 0.26 ml (2.7 mmol) of boron tribromide (BBr₃) over a period of ca. 1 minute. The reaction mixture was stirred for 1 hour, the dry ice/acetone bath was replaced with an ice bath and the mixture was stirred for 1 hour. To the system were added slowly 10 ml of saturated aqueous sodium bicarbonate followed by 10 ml of water, and the mixture was warmed to room temperature. The mixture was extracted with two portions of dichloromethane, and the extracts were dried (Na₂SO₄) and concentrated. The resulting oil (280 mg) was dissolved in CH₂Cl₂, and the solution was extracted with two portions of 1M aqueous NaOH. The combined aqueous extracts were acidified with 2M aqueous HCI and extracted with three portions of dichloromethane. These dichloromethane extracts were dried (Na₂SO₄) and concentrated to obtain 200 mg of the title compound.

¹H NMR (CDCl₃) 6.96 (d, 1H, J=9), 7.36 (m, 2H), 9.84 (s, 1H), 10.9 (s, 1H).

### B. (2S,3S)-3-(2-Hydroxy-5-trifluoromethoxybenzyl)-amino-2-phenylpiperidine Hydrochloride

The title compound was prepared in a manner similar to the compound of Example 4 by replacing 2-methoxy-5-trifluoromethoxybenzaldehyde with 2-hydroxy-5-trifluoromethoxybenzaldehyde.

¹H NMR (free base, CDCl₃) 1.60 (m, 3H), 2.04 (m, 1H), 2.76 (m, 1H), 2.88 (m, 1H), 3.18 (m, 1H), 3.42 (s, 2H), 3.90 (m, 1H), 6.52 (m, 1H), 6.64 (d, 1H, J=9), 6.89 (m, 1H), 7.30 (m, 5H).

HRMS calc'd for C₁₉H₂₁F₃N₂O₂: 366.1545. Found: 366.1562.

Anal. calc'd for C₁₉H₂₁F₃N₂O₂•2HCl•1/3H₂O: C, 51.25; H, 4.90; N, 6.29. Found: C, 51.30; H, 4.75; N, 6.22.

### EXAMPLE 8

### (2S,3S)-3-(5-Chloro-2-[2.2.2-trifluoroethoxy]benzyl)-amino-2-phenylpiperidine Hydrochloride

### A. 5-Chloro-2-(2,2,2-trifluoroethoxy)benzaldehyde

Under a nitrogen atmosphere, in a round-bottom flask were placed 880 mg (22 mmol) of 60% sodium hydride (NaH) and 12 ml of N,N-dimethylformamide. To the system were added 2.9 ml (4 g, 40 mmol) of 2,2,2-trifluoroethanol via syringe over a period of 15 minutes and the mixture was stirred at room temperature for 20 minutes. To the system were added 1.72 g (10 mmol) of 2,5-dichlorobenzonitrile, and the mixture was heated at 90°C for three days. The mixture was cooled to room temperature, poured into 50 ml of 2M aqueous HCI and extracted with three portions of ether. The combined organic fractions were dried (Na₂SO₄) and concentrated to afford 2.5 g of a solid. The crude material was purified by flash column chromatography using 1:49 ethyl acetate/hexanes as the eluant to obtain 1.4 g of 5-chloro-2-(2,2,2-trifluoroethoxy)benzonitrile as a white solid.

M.p. 61-62°C.

Under a nitrogen atmosphere, in a round-bottom flask equipped with a reflux condenser were placed 400 mg (1.7 mmol) of the above nitrile and 10 ml of formic acid. To the system were added ca. 500 mg of Raney nickel and the mixture was heated at reflux for 6 hours and stirred at room temperature overnight. The mixture was filtered through a pad of a diatomaceous earth, and the pad was rinsed with water and CHCl₃. The layers were separated and the aqueous phase was extracted with three portions of CHCl₃. The combined organic fractions were dried and concentrated to obtain 270 mg of the title compound.

¹H NMR (CDCl₃) 4.42 (m, 2H), 6.86 (d, 1H, J=10), 7.46 (m, 1H), 7.80 (d, 1H, J=3), 10.3 (s, 1H).

Mass spectrum: m/z 238 (parent).

### B. (2S,3S)-3-(5-Chloro-2-[2,2,2-trifluoroethoxy]-benzyl)amino-2-phenylpiperidine Hydrochloride

The title compound was prepared in a manner similar to the procedure described in Example 4 by replacing 2-methoxy-5-trifluoromethoxybenzaldehyde with 5-chloro-2-(2,2,2-trifluoroethoxy)benzaldehyde.

M.p. 267-269°C.

¹H NMR (free base, CDCl₃) 1.4 (m, 1H), 1.6 (m, 1H), 1.82 (m, 1H), 2.02 (m, 1H), 2.78 (m, 2H), 3.2 (m, 1H), 3.3 (d, 1H, J=15), 3.54 (d, 1H, J=15), 3.84 (d, 1H, J=3), 4.0 (m, 2H), 6.54 (d, 1H, J=10), 6.92 (d, 1H, J=3), 7.04 (m, 1H), 7.24 (m, 5H).

Anal. calc'd for C₂₀H₂₂ClF₃N₂O•2HCl: C, 50.91; H, 5.13; N, 5.94. Found: C, 50.89; H, 4.84; N, 5.93.

### EXAMPLE 9

### (2S,3S)-2-Phenyl-3-(3-trifluoromethoxybenzyl)-aminopiperidine Hydrochloride

The title compound was prepared in a manner similar to the procedure described in Example 4 by replacing 2-methoxy-5-trifluoromethoxybenzaldehyde with 3-trifluoromethoxybenzaldehyde.

M.p. > 275°C.

¹H NMR (free base, CDCl₃) 1.4 (m, 1H), 1.56 (m, 1H), 1.78 (m, 1H), 1.96 (m, 1H), 2.76 (m, 2H), 3.18 (m, 1H), 3.30 (d, 1H, J=15), 3.46 (d, 1H, J=15), 3.84 (d, 1H, J=3), 6.79 (s, 1H), 6.85 (d, 1H, J=6), 6.94 (m, 1H), 7.12 (m, 1H), 7.24 (m, 5H).

Anal. calc'd for C₁₉H₂₁F₃N₂O•2HCl: C, 53.91; H, 5.48; N, 6.62. Found: C, 53.84; H, 5.07; N, 6.59.

The title compounds of Examples 10-23 and 26 were prepared in a manner similar to the procedure described in Example 4, by replacing 2-methoxy-5-trlfluoromethoxybenzaldehyde with the appropriate aldehyde. Reaction sequences for the preparation of the requisite aldehydes are set forth in Table 1 below.

**TABLE 1**

| Preparation of Compounds of the Formula XII | | |
|---|---|---|
| -C₆H₂X₁X₂X₃ | Starting Material | Reaction*Sequence |
| 2-(2,2,2-trifluoroethoxy)phenyl | 2-chlorobenzonitrile | d,e |
| 2-hydroxy-5-trifluoromethoxyphenyl | 2-methoxy-5-trifluoromethoxybenzaldehyde | f |
| 3-trifluoromethoxyphenyl | - | commercial |
| 5-chloro-2-(2,2,2-trifluoroethoxy)-phenyl | 2,5-dichlorobenzonitrile | d, e |
| 5-t-butyl-2-trifluoromethoxyphenyl | trifluoromethoxybenzene | g, h |
| 2-ethoxy-5-trifluoromethoxyphenyl | 4-trifluoromethoxyphenol | i, a |
| 2-difluoromethoxy-5-trifluoro-methoxyphenyl | 2-hydroxy-5-trifluoromethoxybenzaldehyde | j |
| 5-isopropyl-2-(2,2,2-trifluoroethoxy)phenyl | 4-isopropyl-iodobenzene | d, a |
| 2-isopropoxy-5-trifluoromethoxy-phenyl | 4-trifluoromethoxyphenol | k, a |
| 5-t-butyl-2-difluoromethoxyphenyl | 4-t-butylphenol | a,j |
| 2,5-bis(difluoromethoxy)phenyl | 2,5-dihydroxybenzaldhyde | j |
| 2-difluoromethoxy-5-dimethylamino-phenyl | 5-amino-2-hydroxybenzaldehyde | l, j |
| 2-difluoromethoxy-5-isopropylphenyl | 4-isopropylphenol | a,j |
| 2-difluoromethoxy-5-nitrophenyl | 2-hydroxy-5-nitrobenzaldehyde | j |
| 5-dimethylamino-2-(2,2,2-trifluoro-ethoxy)phenyl | 2-chloro-5-nitrobenzonitrile | d, l, e |
| 5-acetamido-2-(2,2,2-trifluoro-ethoxy)phenyl | 5-nitro-2-(2,2,2-trifluoroethoxy)-benzonitrile | m, c, e |
| 2-difluoromethoxy-5-ethylphenyl | 4-ethyl-methoxybenzene | a, f,j |
| 5-chloro-2-difluoromethoxyphenyl | 5-chloro-2-hydroxybenzaldehyde | j |
| 2-trifluoromethoxyphenyl | - | commercial |
| 2-methoxy-5-trifluoromethoxyphenyl | 4-trifluoromethoxyphenol | b, a |
| 2-difluoromethoxy-5-methylphenyl | 5-methyl-2-methoxybenzaldehyde | f, j |

| | | |
|---|---|---|
| *Reagents for Preparation of Compounds of the Formula XII | | |
| From Standard Routes a) C1₂CHOCH₃, TiC1₄ | | |
| b) methyl iodide | | |
| c) acetyl chloride | | |
| d) NaOCH₂CF₃ | | |
| e) Raney nickel, HCO₂H | | |
| f) BBr₃ | | |
| g) t-butyl chloride/A1C1₃ | | |
| h) C1₂CHOCH₃/A1C1₃ | | |
| i) ethyl iodide | | |
| j) ClF₂CH | | |
| k) isopropyl bromide | | |
| l) H₂, Pd/C, HCHO | | |
| m) H₂-Pd/BaSO₄ | | |

### EXAMPLE 10

### (2S,3S)-3-[5-Chloro-2-(2,2,2-trifluoroethoxy)benzyl]-amino-2-phenylpiperidine hydrochloride

M.P. 267-269°C.

¹H NMR (free base; CDCl₃) 1.40 (m, 1H), 1.60 (m, 1H), 1.82 (m, 1H), 2.02 (m, 1H), 2.76 (m, 2H), 3.20 (m, 1H), 3.28 (d, 1H, J=15), 3.52 (d, 1H, J=15), 3.84 (d, 1H, J=3), 4.00 (m, 2H), 6.54 (d, 1H, J=10), 6.92 (d, 1H, J=3), 7.04 (m, 1H), 7.24 (m, 5H).

HRMS calc'd for C₂₀H₂₂ClF₃N₂O: 398.1368. Found: 398.1352.

Anal. calc'd for C₂₀H₂₂ClF₃N₂O•2HCl: C, 50.91; H, 5.13; N, 5.94. Found: C, 50.89; H, 4.84; N, 5.93.

### EXAMPLE 11

### (2S,3S)-3-(5-t-Butyl-2-trifluoromethoxybenzvl)amino-2-phenylpiperidine hydrochloride

M.P. 262-264°C.

¹H NMR (free Base; CDCl₃) 1.20 (s, 9H), 1.40 (m, 1H), 1.52 (m, 1H), 1.84 (m, 1H), 2.06 (m, 1H), 2.80 (m, 2H), 3.22 (m, 1H), 3.38 (d, 1H, J=15), 3.58 (d, 1H, J=15), 3.86 (d, 1H, J=3), 6.98 (m, 1H), 7.12 (m, 2H), 7.26 (m, 5H).

HRMS calc'd for C₂₃H₂₉F₃N₂O: 406.2225. Found: 406.2271.

Anal. calc'd for C₂₃H₂₉F₃N₂O•2HCl•1/3H₂O: C, 56.92; H, 6.56; N, 5.77. Found: C, 56.99; H, 6.41; N, 6.03.

### EXAMPLE 12

### (2S,3S)-3-[5-Isopropyl-2-(2,2,2-trifluoroethoxy)-benzyl]amino-2-phenylpiperidine hydrochloride

M.P. > 280°C.

¹H NMR (free base; CDCl₃) 1.12 (m, 6H), 1.4 (m, 1H), 1.62 (m, 1H), 1.82 (m, 1H), 2.08 (m, 1H), 2.76 (m, 3H), 3.22 (m, 1H), 3.30 (d, 1H, J=15), 3.38 (d, 1H, J=15), 3.82 (d, 1H, J=3), 4.02 (m, 2H), 6.56 (d, 1H, J=10), 6.78 (d, 1H, J=3), 6.94 (m, 1H), 7.24 (m, 5H).

HRMS calc'd for C₂₃H₃₀F₃N₂O (M+1): 407.2303. Found: 407.2287.

Anal. calc'd for C₂₃H₂₉F₃N₂O•2HCl•1/2H₂O: C, 56,55, H, 6.60; N, 5.70. Found: C, 56.17: H, 6.39; N, 5.77.

### EXAMPLE 13

### (2S,3S)-3-[5-Dimethylamino-2-(2,2,2-trifluoroethoxy)-benzyl]amino-2-phenylpiperidine hydrochloride.

M.P. 250-252°C.

¹H NMR (free base; CDCl₃) 1.40 (m, 1H), 1.60 (m, 1H), 1.86 (m, 1H), 2.10 (m, 1H), 2.82 (m, 8H), 3.22 (m, 1H), 3.34 (d, 1H, J=15), 3.58 (d, 1H, J=15), 3.88 (d, 1H, J=3), 4.00 (m, 2H), 6.42 (d, 1H, J=3), 6.50 (m, 1H), 6.64 (d, 1H, J=10), 7.30 (m, 5H).

HRMS calc'd for C₂₂H₂₈F₃N₃O: 407.2178. Found: 407.2179.

### EXAMPLE 14

### (2S,3S)-3-(2-Difluoromethoxy-5-N,N-dimethylamino-benzyl)amino-2-phenylpiperidine hydrochloride

M.P. 243-245°C (dec).

¹H NMR (free base; CDCl₃) 1.44 (m, 1H), 1.72 (m, 2H), 2.10 (m, 1H), 2.84 (m, 8H), 3.21 (m, 1H), 3.28 (d,1H, J=15), 3.55 (d, 1H, J=15), 3.88 (d, 1H, J=3), 6.08 (t, 1H, J=72), 6.36 (d, 1H, J=3), 6.46 (dd, 1H, J=3,9), 6.86 (d, 1H, J=9), 7.28 (m, 5H).

HRMS calc'd for C₂₁H₂₇F₂N₃O: 375.2122. Found: 375.2138.

Anal. calc'd for C₂₁H₂₇F₂N₃O•3HCl•1/2H₂O: C, 51.07; H, 6.44; N, 8.51. Found: C, 50.71; H, 6.08; N, 8.28.

### EXAMPLE 15

### (2S,3S)-3-[2,5-bis(difluoromethoxy)benzyl]amino-2-phenylpiperidine hydrochloride

M.P. 238-239°C.

¹H NMR (free base; CDCl₃) 1.64 (m, 3H), 2.04 (m, 1H), 2.76 (m, 2H), 3.18 (m, 1H), 3.28 (d, 1H, J=12), 3.52 (d, 1H, J=12), 3.84 (d, 1H, J=3), 6.12 (t, 1H, J=75), 6.40 (t, 1H, J=75), 6.75 (m, 2H), 6.94 (d, 1H, J=9), 7.24 (m, 5H).

HRMS calc'd for C₂₀H₂₂F₄N₂O₂: 398.1612. Found: 398.1591.

### EXAMPLE 16

### (2S,3S)-3-(5-t-Butyl-2-difluoromethoxybenzyl)amino-2-phenylpiperidinehydrochloride

M.P. 263-264°C (dec).

¹H NMR (free base; CDCl₃) 1.24 (s, 9H), 1.42 (m, 1H), 1.62 (m, 1H), 1.80 (m, 1H), 2.10 (m, 1H), 2.80 (m, 2H), 3.24 (m, 2H), 3.58 (d, 1H, J=12), 3.87 (brs, 1H), 6.18 (t, 1H, J=72), 6.86 (d, 1H, J=6), 7.00 (brs, 1H), 7.12 (m, 1H), 7.24 (m, 5H).

HRMS calc'd for C₂₃H₃₀F₂N₂O: 388.2321. Found: 388.2336.

### EXAMPLE 17

### (2S,3S)-3-(2-Isopropoxy-5-trifluoromethoxybenzyl)amino-2-phenylpiperidine hydrochloride

M.P. 245-246°C (dec).

¹H NMR (free base: CDCl₃) 1.08 (d, 3H, J=6), 1.12 (d, 3H, J=6), 1.40 (m, 1H), 1.64 (m, 1H), 1.87 (m, 1H), 2.08 (m, 1H), 2.78 (m, 2H), 3.02 (m, 1H), 3.34 (d, 1H, J=15), 3.51 (d, 1H, J=15), 3.85 (d, 1H, J=2), 4.28 (m, 1H), 6.01 (d, 1H, J=9), 6.82 (m, 1H), 6.91 (m, 1H), 7.24 (m, 5H).

HRMS calc'd for C₂₂H₂₇F₃N₂O₂: 408.2024. Found: 408.2019.

Anal. calc'd for C₂₂H₂₇F₃N₂O₂•2HCl: C, 54.89; H, 6.07, N, 5.82. Found: C, 54.50; H, 6.24; N, 5.78.

### EXAMPLE 18

### (2S,3S)-3-(2-Difluoromethoxy-5-trifluoromethoxybenzyl)-amino-2-phenylpiperidine hydrochloride

M.P. 257-259°C (dec).

¹H NMR (free base; CDCl₃) 1.44 (m, 1H), 1.58 (m, 1H), 1.78 (m, 1H), 2.03 (m, 1H), 2.78 (m, 2H), 3.20 (m, 1H), 3.32 (d, 1H, J=15), 3.54 (d, 1H, J=15), 3.87 (d, 1H, J=2), 6.15 (t, 1H, J=72), 6.94 (m, 3H), 7.26 (m, 5H).

HRMS calc'd for C₂₀H₂₁F₅N₂O₂: 416.1523. Found: 416.1501.

Anal. calc'd for C₂₀H₂₁F₅N₂O₂•2HCl•1/3H₂O: C, 48.50; H, 4.81; N, 5.65. Found: C, 48.45; H, 4.57; N, 5.66.

### EXAMPLE 19

### (2S,3S)-3-(2-Ethoxy-5-trifluoromethoxybenzyl)amino-2-phenylpiperidine hydrochloride

M.P. > 275°C (dec).

¹H NMR (free base; CDCl₃) 1.13 (t, 3H, J=6), 1.38 (m, 1H), 1.70 (m, 2H), 2.06 (m, 1H), 2.74 (m, 2H), 3.22 (m, 1H), 3.30 (d, 1H, J=15), 3.68 (m, 3H), 3.84 (br s, 1H), 6.55 (d, 1H, J=9), 6.79 (br s, 1 H), 6.90 (m, 1 H), 7.2 (m, 5H).

HRMS calc'd for C₂₁H₂₅F₃N₂O₂: 394.1868. Found: 394.1875.

Anal. calc'd for C₂₁H₂₅F₃N₂O₂•2HCl: C, 53.97; H, 5.82; N, 6.00. Found: C, 53.85; H, 5.79; N, 5.95.

### EXAMPLE 20

### (2S,3S)-3-(2-Difluoromethoxy-5-nitrobenzyl)amino-2-phenylpiperidine hydrochloride

¹H NMR (free base; CDCl₃) 1.50 (m, 1H), 1.66 (m, 1H), 1.98 (m, 2H), 2.82 (m, 2H), 3.28 (m, 1H), 3.42 (d, 1 H, J=15), 3.64 (d, 1 H, J=15), 3.95 (d, 1H, J=2), 6.30 (t, 1 H, J=72), 7.08 (d, 1H, J=8), 7.30 (m, 5H), 8.04 (m, 2H).

FAB HRMS calc'd for C₁₉H₂₁F₂N₃O₃(M+1): 378.1629. Found: 378.1597.

### EXAMPLE 21

### (2S,3S)-3-(2-Difluoromethoxy-5-isopropylbenzyl)amino-2-phenylpiperidine hydrochloride

M.P. 245-247°C (dec).

¹H NMR (free base; CDCl₃) 1.19 (2d, 6H, J=7), 1.50 (m, 1H), 1.75 (m, 2H), 2.12 (m, 1H), 2.83 (m, 3H), 3.25 (m, 1H), 3.35 (d, 1H, J=14), 3.60 (d, 1H, J=14), 3.90 (d, 1H, J=3), 6.20 (t, 1H, J=75), 6.90 (m, 2H), 7.00 (m, 1H), 7.30 (m, 5H).

HRMS calc'd for C₂₂H₂₈F₂N₂O: 374.2170. Found: 374.2207.

Anal. calc'd for C₂₂H₂₈F₂N₂O•2HCl•1/3H₂O: C, 58.28; H, 6.67; N, 6.18. Found: C, 58.17; H, 6.52; N, 6.17.

### EXAMPLE 22

### (2S,3S)-3-[5-Acetamido-2-(2,2,2-trifluoroethoxy)-benzyl]amino-2-phenylpiperidine hydrochloride

M.P. > 270°C.

¹H NMR (free base; CDCl₃) 1.46 (m, 1H), 1.82 (m, 1H), 2.08 (m, 1H), 2.12 (s, 3H), 2.76 (m, 2H), 3.20 (m, 1H), 3.48 (d, 1H, J=15), 3.58 (d, 1H, J=15), 3.82 (m, 1H), 4.08 (m, 2H), 6.44 (m, 1H), 6.58 (d, 1H, J=10), 6.78 (m, 1H), 7.26 (m, 5H), 7.58 (m, 1H).

### EXAMPLE 23

### (2S,3S)-3-(2-Difluoromethoxy-5-ethylbenzyl)amino-2-phenylpiperidine hydrocholoride

M.P. 254-255°C.

¹H NMR (free base; CDCl₃) 1.12 (t, 3H, J=10), 1.36 (m, 1H), 1.44 (m, 1H), 1.82 (m, 1H), 2.10 (m, 1H), 2.48 (q, 2H, J=10), 2.8 (m, 1H), 3.10 (m, 1H), 3.34 (d, 1H, J=15), 3.58 (d, 1H, J=15), 3.9 (d, 1H, J=3), 6.12 (t, 1H, J=85), 6.78 (s, 1H), 6.90 (m, 2H), 7.28 (m, 5H).

Anal. calc'd for C₂₁H₂₆F₂N₂O•2HCl: C, 58.19; H, 6.51; N, 6.47. Found: C, 57.90; H, 6.52; N, 6.64.

### EXAMPLE 24

### cis-3-(5-t-Butyl-2-methoxybenzyl)amino-2-(3-trifluoro-methoxyphenyl)piperidine hydrochloride

### A. cis-5-Nitro-6-(trifluoromethoxyphenyl)piperidin-2-one

Under a nitrogen atmosphere, in a round-bottom flask were placed 15 g (79 mmol) of 3-trifluoromethoxy-benzaldehyde, 80 mL of ethanol, 11 g (0.26 mol) of ammonium acetate and 12.6 mL (79 mmol) of methyl 4-nitrobutyrate, and the mixture was heated at reflux for 6 hours. After cooling to room temperature, the mixture was concentrated. The remaining material was stirred with ca. 200 mL of CHCl₃ for 30 minutes, filtered and concentrated. The residue purified by flash column chromatography, eluting with 1:49 methanol/chloroform followed by 1:19 methanol/chloroform to obtain 24 g of 5-nitro-6-(3-trifluoromethoxyphenyl)-piperidin-2-one.

In a round bottom flask were placed 20 g (66 mmol) of the product obtained above, 13 g of KOH and 100 mL of ethanol, and the mixture was stirred at room temperature for 90 minutes. To the system was added ca. 35 mL of 33% sulfuric acid/ethanol. The mixture was poured into 150 mL of water and extracted with three 100 mL portions of CHCl₃. The combined extracts were washed with water, dried (Na₂SO₄) and concentrated. The crude material was purified by column chromatography (300 g of silica gel) using ethyl acetate followed by 1:99 methanol/ethyl acetate as the eluant to obtain 5.8 g of cis-5-nitro-6-(3-trifluoromethoxyphenyl)-piperidin-2-one which contained ca. 12% of the corresponding trans-isomer. This material was purified by a second chromatography to obtain 4.6 g of the cis-product.

### B. cis-5-Amino-6-(3-trifluoromethoxyphenyl)piperidin-2-one

Under a nitrogen atmosphere, in a three-neck round-bottom flask equipped with a thermometer and a mechanical stirrer, were placed this cis-material and a mixture of THF (200 mL), methanol (50 mL) and water (5 mL). To this stirring solution was added aluminum amalgam (prepared by washing 4.1 g of aluminum foil strips with ether and dipping in 2% aqueous HgCl₂ for 30-45 seconds and washing with ether), and the mixture was stirred at room temperature overnight. The mixture was filtered through a pad of diatomaceous earth and the pad washed with THF. The filtrate was concentrated, dissolved in ethyl acetate and treated with 30 mL of ether saturated with HCI. Concentration afforded 3.7 g of crude cis-5-amino-6-(3-trifluoromethoxyphenyl)piperidin-2-one as a waxy solid, m.p. 126-130°C.

### C. cis-3-(5-t-Butyl-2-methoxybenzyl)amino-2-(3-trifluoromethoxyphenyl)piperidine hydrochloride

Under a nitrogen atmosphere, in a round-bottom flask were placed 0.38 g (1.4 mmol) of the amine obtained above, 6 mL of acetic acid and 0.32 g (1.66 mmol) of 5-t-butyl-2-methoxybenzaldehyde. The mixture was stirred for 45 minutes. To the system was added 0.65 g (3.0 mmol) of sodium triacetoxyborohydride in portions, and the mixture was stirred at room temperature overnight. The mixture was concentrated and partitioned between chloroform and H₂O and made basic with 1N aqueous NaOH. The layers were separated and the aqueous phase was extracted with two portions of CHCl₃. The combined organic fractions were washed with H₂O, dried and concentrated. The crude product was purified by flash column chromatography to obtain 0.4 g of cis-5-(5-t-butyl-2-methoxybenzyl)amino-6-(3-trifluoromethoxyphenyl)-piperidin-2-one.

Under a nitrogen atomsphere, in a round-bottom flask were placed 0.4 g (0.9 mmol) of the product obtained above and 10 mL of THF. To the system was added 2.2 mL (4.4 mmol) of 2M borane methyl sulfide complex in THF and the mixture was gradually heated and allowed to reflux for 4 hours. The mixture was cooled to room temperature, 2 mL of methanol was added to the system and the mixture was concentrated. To the system was added 5 mL of ethanol and 2.45 of K₂CO₃, and the mixture was heated at reflux for 8 hours and stirred at room temperature overnight. The mixture was concentrated and partitioned between water and CH₂Cl₂. The layers were separated, and the aqueous phase was extracted with three portions of CH₂Cl₃. The combined organic fractions were dried and concentrated to obtain an oil. The oil was dissolved in ethyl acetate, and the solution was treated with ether saturated with HCI. Concentration afforded 70 mg of the title compound as a waxy solid.

M.P. 247°C-249°C.

¹H NMR (free-base, CDCl₃) 1.26 (s, 9H), 1.6 (m, 1H), 1.90 (m, 2H), 2.12 (m, 1H), 2.80 (m, 2H), 3.24 (m, 1H), 3.36 (d, 1H, J=15), 3.48 (s, 3H), 3.64 (d, 1H, J=15), 3.86 (m, 1H), 6.60 (d, 1H,J=10),7.18(m,6H).

HRMS Calc'd: C₂₄H₃₁N₂O₂F₃: 436.2330. Found: 436.2326.

### EXAMPLE 25

### cis-2-(3,5-Dibromophenyl)-3-(2-methoxy-5-trifluoro-methoxybenzyl)aminopiperidine

The title compound was prepared by a procedure similar to that described in Example 25, with the exception that the nitro substituent of the product of the initial reaction [6-(3,5-dibromophenyl)-5-nitropiperidin-2-one] was converted to an amino group by sequential oxidative cleavage (O₃, KO⁺Bu), oxime formation (H₂NOH) and Raney nickel-catalyzed reduction. The final product can be resolved by treatment with (R)-(-)-mandelic acid in isopropanol. Two recrystallizations of the solid isolated from this procedure (isopropanol), followed by treatment with saturated aqueous sodium bicarbonate affords the (2S,3S)-enantiomer; [ ]_{D} (mandelate salt): +4.11°(MeOH, c=0.51).

¹H NMR (CDCl₃) 1.36 (m, 1H), 1.50 (m, 1H), 1.80 (m, 1H), 2.04 (m, 1H), 2.70 (m, 2H), 3.18 (m, 1H), 3.30 (d, 1H, J=18), 3.57 (s, 3H), 3.66 (d, 1H, J=18), 3.75 (m, 1H), 6.63 (d, 1H, J=9), 6.86 (d, 1H, J=3), 6.97 (dd, 1H, J=6, 9), 7.32 (m, 2H), 7.48 (s, 1H).

### EXAMPLE 26

### (2S-3S)-3-(2-Difluoromethoxy-5-methylbenzyl)amino-2-phenylpiperidinehydrochloride

The title compound was prepared by a procedure similar to that described in Example 4. M.P. >275°C.

¹H NMR (free-base, CDCl₃) 1.44 (m, 1H), 1.6 (m, 1H), 1.84 (m, 1H), 2.10 (m, 1H), 2.20 (s, 3H), 2.80 (m, 2H), 3.22 (m, 1H), 3.34 (d, 1H, J=15), 3.58 (d, 1H, J=15), 3.90 (d, 1H, J=3), 6.10 (t, 1H, J=72), 6.84 (m, 2H), 7.26 (m, 5H).

HRMS calc'd for C₂₀H₂₄F₂N₂O: 347.1929 (M+1). Found: 347.1911.

Anal. calc'd for C₂₀H₂₄F₂N₂O•2HCl•0.25H₂O: C, 56.67; H, 6.30; N, 6.61. Found: C, 56.81; H, 6.16; N, 6.50.

### Aspects of the Invention:

1. A method of treating a disorder or condition selected from sleep disorders; autism; pervasive development disorder; rheumatoid arthritis; osteoarthritis; fibromyalgia; human immunodeficiency virus (HIV) infections; dissociative disorders such as body dysmorphic disorders; eating disorder such as anorexia and bulimia; ulcerative colitis; Crohn's disease; irritable bowel syndrome; functional abdominal pain; chronic fatigue syndrome; sudden infant death syndrome (SIDS); overactive bladder; chronic cystitis; chemotherapy induced cystitis; cough, angiotensin converting enzyme (ACE) induced cough; itch; hiccups; premenstrual syndrome: premenstrual dysphoric disorder; schizophrenia; schizoaffective disorder; delusional disorder; substance-induced psychotic disorder; brief psychotic disorder; shared psychotic disorder; psychotic disorder due to a general medical condition; schizophreniform disorder; amenorrheic disorders such as dysmenorrhea; obesity; epilepsy: movement disorders such as primary movement disorders, spasticities, Scott's syndrome, Tourette's syndrome, palsys, amyolateral sclerosis (ALS), akinetic-rigid disorders, akinesias, dyskinesias, restless leg syndrome and movement disorders associated with Parkinson's disease or Huntington's disease; mastalgia syndromes; motion sickness; immune dysfunctions; generalized anxiety disorder; panic disorder; phobias, including social phobia, agoraphobia, and specific phobias; obsessive-compulsive disorder; post-traumatic stress disorder; depression including major depression, single episode depression, recurrent depression, child abuse induced depression, postpartum depression and dysthemia; cyclothymia; bipolar disorder; neurocardiac disorders such as neurocardiac syncope, neurogenic syncope, hypersensitive Carotid sinus, neurovascular syndrome and arrythmias including arrythmias secondary to gastrointestinal disturbances; addiction disorders involving addictions to behaviors; HIV-1 associated dementia, AIDS dementia complex, HIV encephalopathy, HIV related neuralgias; AIDS related neuralgias; epilepsy; and attention deficit hyperactivity disorder in a mammal, comprising administering to said mammal an amount of a compound of the formula I, 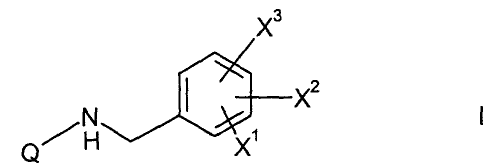 wherein X¹ is hydrogen, (C₁-C₁₀) alkoxy optionally substituted with from one to three fluorine atoms or (C₁-C₁₀) alkyl optionally substituted with from one to three fluorine atoms;
   X² and X³ are independently selected from halo, hydrogen, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, -C(=O)-NH-(C₁-C₆)alkyl, (C₁-C₆)alkyl-C(=O)-NH-(C₁-C₆)alkyl, hydroxy(C₁-C₄)alkyl, (C₁-C₄)alkoxy(C₁-C₄)alkyl, -NHC(=O)H and -NHC(=O)-(C₁-C₆)alkyl; and
   Q is a group of the formula 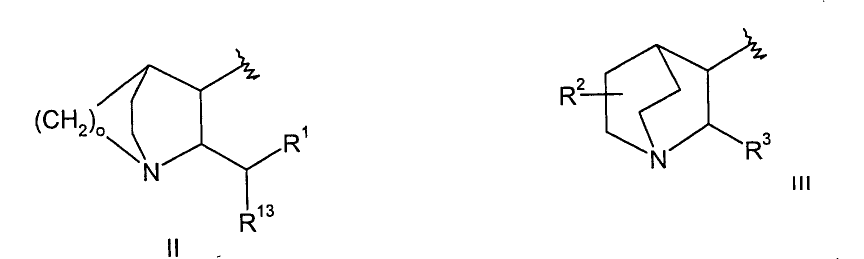 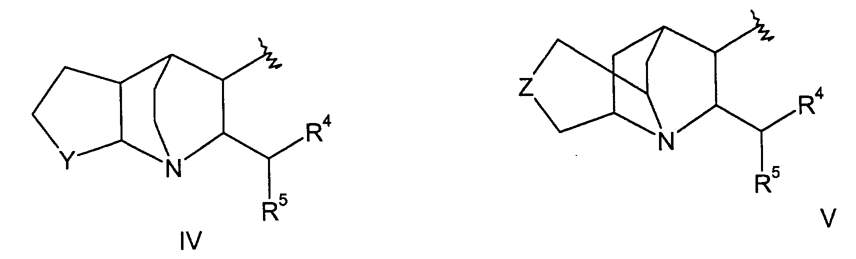 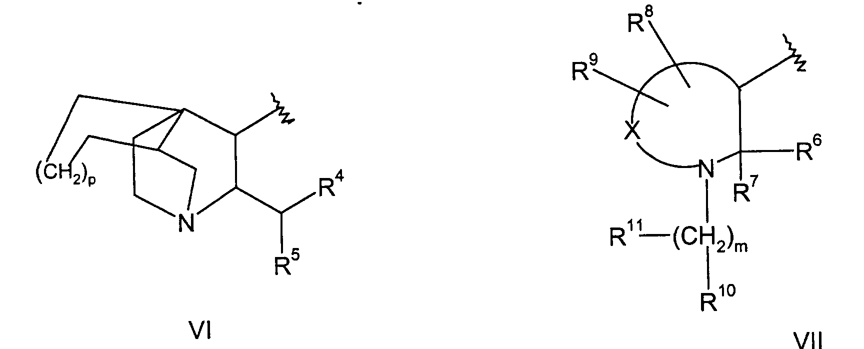 OR 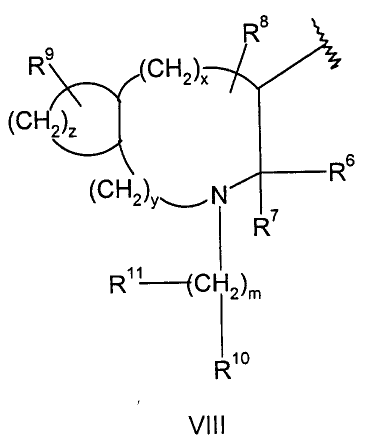 wherein R¹ is a radical selected from furyl, thienyl, pyridyl, indolyl, biphenyl and phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
   R¹³ is selected from (C₃-C₄) branched alkyl, (C₅-C₆) branched alkenyl, (C₅-C₇) cycloalkyl, and the radicals named in the definition of R¹;
   R² is hydrogen or (C₁-C₆) alkyl;
   R³ is phenyl, biphenyl, naphthyl, pyridyl, benzhydryl, thienyl or furyl, and R³ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
   Y is (CH₂)₁ wherein I is an integer from one to three, or Y is a group of the formula 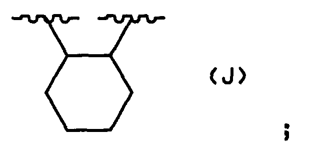
   Z is oxygen, sulfur, amino, (C₁-C₃)alkylamino or (CH₂)ₙ wherein n is zero, one or two;
   o is two or three;
   p is zero or one;
   x is an integer from zero to four;
   y is an integer from zero to four;
   z is an integer from one to six, and the ring in formula VIII containing (CH₂)_{z} may contain from zero to three double bonds, and one of the carbons of said (CH₂)_{z} may optionally be replaced by oxygen, sulphur or nitrogen;
   R⁴ is furyl, thienyl, pyridyl, indolyl, biphenyl, or phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, (C₁-C₃) alkoxy-carbonyl and benzyloxycarbonyl;
   R⁵ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
   X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R⁸, and wherein any one of the carbon atoms of said (CH₂)_{q} may optionally be substituted with R⁹;
   m is an integer from 0 to 8, and any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R¹¹;
   R⁶ is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆) alkyl-O-C(=O)- (C₁-C₆)alkyl, (C₁-C₆)alkyl-C(=O)-O-, (C₁-C₆)alkyl-C(=O)- (C₁-C₆)alkyl-O-, (C₁-C₆)alkyl-C(=O)-, (C₁-C₆)alkyl-C(=O)- (C₁-C₆)alkyl-, di-(C₁-C₆)alkylamino, -C(=O)NH-(C₁-C₆)alkyl,(C₁-C₆)-alkyl-C(=O)-NH-(C₁-C₆)alkyl, -NHC(=O)H and -NHC(=O)-(C₁-C₆) alkyl; and wherein one of the phenyl moieties of said benzhydryl may optionally be replaced by naphthyl, thienyl, furyl or pyridyl;
   R⁷ is hydrogen, phenyl or (C₁-C₆)alkyl;
   or R⁶ and R⁷, together with the carbon to which they are attached, form a saturated carbocyclic ring having from 3 to 7 carbon atoms wherein one of said carbon atoms may optionally be replaced by oxygen, nitrogen or sulfur;
   R⁸ and R⁹ are each independently selected from hydrogen, hydroxy, halo, amino, oxo (=O), nitrile, hydroxy-(C₁-C₆)-alkyl, (C₁-C₆)alkoxy-(C₁-C₆)alkyl, (C₁-C₆)alkylamino, di-(C₁-C₆)alkylamino, (C₁-C₆)alkoxy, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C-, (C₁-C₆)alkyl-C(=O)-(C₁-C₆)alkyl-, and the radicals set forth in the definition of R⁶;
   R¹⁰ is NHC(=O)R¹², NHCH₂R¹², NHSO₂R¹² or one of the radicals set forth in any of the definitions of R⁶, R⁸ and R⁹;
   R¹¹ is oximino (=NOH) or one of the radicals set forth in any of the definitions of R⁶, R⁸ and R⁹; and
   R¹² is (C₁-C₆)alkyl, hydrogen, phenyl(C₁-C₆)alkyl or phenyl optionally substituted with (C₁-C₆)alkyl;
   with the proviso that (a) when m is 0, R¹¹ is absent, (b) neither R⁸, R⁹, R¹⁰ nor R¹¹ can form, together with the carbon to which it is attached, a ring with R⁷, (c) when Q is a group of the formula VIII, R⁸ and R⁹ cannot be attached to the same carbon atom, (d) when R⁸ and R⁹ are attached to the same carbon atom, then either each of R⁸ and R⁹ is independently selected from hydrogen, fluoro, (C₁-C₆) alkyl, hydroxy-(C₁-C₆)alkyl and (C₁-C₆)alkoxy-(C₁-C₆)alkyl, or R⁸ and R⁹, together with the carbon to which they are attached, form a (C₃-C₆) saturated carbocyclic ring that forms a spiro compound with the nitrogen-containing ring to which they are attached, (e) when neither X¹, X² nor X³ is a fluorinated alkoxy group, at least one of R¹, R³, R⁴, R⁵, R⁶, R⁷ and R¹³ is an aryl group substituted with a fluorinated alkoxy group;
   or a pharmaceutically acceptable salt thereof,
   or a compound selected from the group consisting of:
      (2S,3S)-3-(6-methoxy-3-trifluoromethyl-1,3-dihydroisobenzofuran-5-yl)methylamino-2-phenylpiperidine;
      (2S,3S)-3-(6-methoxy-1-methyl-1-trifluoromethylisochroman-7-yl)methylamino-2-phenylpiperidine;
      (2S,3S)-3-(6-methoxy-3-methyl-3-trifluoromethyl-1,3-dihydroisobenzofuran-5-yl)methylamino-2-phenylpiperidine;
      (2S,3S)-3-(6-methoxy-3-phenyl-3-trifluoromethyl-1,3-dihydroisobenzofuran-5-yl)methylamino-2-phenylpiperidine;
      (2S,3S)-3-[1-(6-methoxy-3-methyl-3-trifluoromethyl-1,3-dihydroisobenzofuran-5-yl)ethylamino]-2-phenylpiperidine;
      (2S,3S)-3-[(1R)-6-methoxy-1-methyl-1-trifluoromethylisochroman-7-yl]methylamino-2-phenylpiperidine;
      (2S,3S)-3-[(3R)-6-methoxy-3-methyl-3-trifluoromethyl-1,3-dihydroisobenzofuran-5-yl)methylamino-2-phenylpiperidine;
      (2S,3S)-N-(5-ethyl-2-methoxyphenyl)methyl-2-diphenylmethyl-1-azabi-cyclo[2.2.2]-octan-3-amine;
      (2S,3S)-N-(5-isopropyl-2-methoxyphenyl)methyl-2-di-phenylmethyl-1-azabicyclo[2.2.2]-octan-3-amine;
      (2S,3S)-N-(5-sec-butyl-2-methoxyphenyl)-methyl-2-diphenylmethyl-1-azabicyclo[2.2.2]-octan-3-amine;
      (2S,3S)-N-(5-tert-butyl-2-methoxyphenyl)-methyl-2-diphenylmethyl-1-azabicyclo[2.2.2]-octan-3-amine; and
      (2S,3S)-N-(5-methyl-2-methoxyphenyl)methyl-2-diphenylmethyl-1-azabicyclo[2.2.2]-octan-3-amine;
      or a pharmaceutically acceptable salt thereof, that is effective in treating such disorder or condition.
2. A method according to aspect 1, wherein the compound of formula I that is employed in such method is selected from the following compounds and their pharmaceutically acceptable salts:
   2-(diphenylmethyl)-N-((2-difluoromethoxy)-phenyl)methyl-1-azabicyclo[2.2.2]octan-3-amine;
   (2S,3S)-N-(2-methoxy-5-trifluoromethoxy-phenyl)methyl-2-diphenylmethyl-1-azabicyclo[2.2.2]octane-3-amine;
   (2S,3S)-2-phenyl-3-[2-(2,2,2-trifluoroethoxy)-benzyl]aminopiperidine;
   (2S,3S)-3-(2-methoxy-5-trifluoromethoxybenzyl)-amino-2-phenylpiperidine;
   (2S,3S)-3-(2-hydroxy-5-trifluoromethoxybenzyl)-amino-2-phenylpiperidine;
   (2S,3S)-2-phenyl-3-(3-trifluoromethoxybenzyl)-aminopiperidine;
   (2S,3S)-1-(5,6-dimethoxyhexyl)-3-(2-methoxy-5-trifluoromethoxybenzyl)amino-2-phenylpiperidine;
   (2S,3S)-2-phenyl-3-(2-trifluoromethoxybenzyl)-aminopiperidine;
   (2S,3S)-3-[5-chloro-2-(2,2,2-trifluoroethoxy)-benzyl]amino-2-phenylpiperidine;
   (2S,3S)-3-(5-t-butyl-2-trifluoromethoxy-benzyl)amino-2-phenylpiperidine;
   3-(5-tert-butyl-2-methoxybenzyl)amino-2-(3-trifluoromethoxyphenyl)plperidine;
   3-(2-isopropoxy-5-trifluoromethoxybenzyl)amino-2-phenyl)piperidine; and
   3-(2-difluoromethoxy-5-trifluoromethoxybenzyl)-amino-2-phenylpiperidine.
3. A method according to aspect 1, wherein the disorder or condition being treated is cyclothymia or bipolar disorder.
4. A method according to aspect 1, wherein the disorder or condition being treated is an addiction to a behavior.
5. A method according to aspect 1, wherein the disorder or condition being treated is a sleep disorder.
6. A method according to aspect 1, wherein the disorder or condition being treated is premenstrual syndrome or premenstrual dysphoric disorder.
7. A method according to aspect 1, wherein the disorder or condition being treated is autism or pervasive development disorder.
8. A method according to aspect 1, wherein the disorder or condition being treated is Scott's syndrome or Tourette's syndrome.
9. A method according to aspect 1, wherein the disorder or condition being treated is obsessive-compulsive disorder.
10. A method according to aspect 1, wherein the disorder or condition being treated is selected from movement disorders such as primary movement disorders, spasticities, Scott's syndrome, Tourette's syndrome, palsys, amyolateral sclerosis (ALS), akinetic-rigid disorders, akinesias, dyskinesias, restless leg syndrome and movement disorders associated with Parkinson's disease or Huntington's disease.
11. A method according to aspect 1, wherein the disorder or condition being treated is major depressive disorder.
12. A method according to aspect 1, wherein the disorder or condition being treated is generalized anxiety disorder.
13. A method according to aspect 1, wherein the disorder or condition being treated is irritable bowel syndrome.
14. A method according to aspect 1, wherein the disorder or condition being treated is functional abdominal pain.
15. A method according to aspect 1, wherein the disorder or condition being treated is an HIV infection.
16. A method according to aspect 1, wherein the disorder or condition being treated is an immune dysfunction.
17. A method according to aspect 1, wherein the disorder or condition being treated is selected from neurocardiac disorders such as neurocardiac syncope, neurogenic syncope, hypersensitive Carotid sinus, neurovascular syndrome and arrythmias including arrythmias secondary to gastrointestinal disturbances.
18. A method according to aspect 1, wherein the disorder or condition being treated is selected from major depression, single episode depression, recurrent depression, child abuse induced depression, postpartum depression, dysthymia, cyclothymia and bipolar disorder.
19. A method according to aspect 1, wherein the disorder or condition being treated is selected from as body dysmorphic disorders and eating disorders such as anorexia and bulimia.
20. A method according to aspect 1, wherein the disorder or condition being treated is selected from schizophrenia, schizoaffective disorder, delusional disorder, substance-induced psychotic disorder, brief psychotic disorder, shared psychotic disorder, psychotic disorder due to a general medical condition, and schizophreniform disorder.
21. A method according to aspect 1, wherein the disorder or condition being treated is selected from premenstrual syndrome, premenstrual dysphoric disorder, and amenorrheic disorders such as dysmenorrhea.
22. A method according to aspect 1, wherein the disorder or condition being treated is selected from Crohn's disease, ulcerative colitis, irritable bowel syndrome and functional abdominal pain.
23. A method according to aspect 1, wherein the disorder or condition being treated is selected from autism, pervasive development disorder, and attention deficit hyperactivity disorder.
24. A method according to aspect 1, wherein the disorder or condition being treated is selected from chronic fatigue syndrome, sudden infant death syndrome (SIDS), obesity, and epilepsy.
25. A method according to aspect 1, wherein the disorder or condition being treated is selected from generalized anxiety disorder, panic disorder, obsessive-compulsive disorder, post-traumatic stress disorder, and phobias, including social phobia, agoraphobia, and specific phobias.
26. A method according to aspect 1, wherein the disorder or condition being treated is selected from cough, angiotensin converting enzyme (ACE) induced cough, itch, and hiccups.
27. A method according to aspect 1, wherein the disorder or condition being treated is selected from overactive bladder; chronic cystitis and chemotherapy induced cystitis.
28. A method according to aspect 1, wherein the disorder or condition being treated is a sleep disorder.
29. A method according to aspect 1, wherein the compound of formula I is administered to a human for the treatment of major depressive disorder and concomitant neuropathic pain.
30. A method according to aspect 1, wherein the compound of formula I is administered to a human for the treatment of any two or more comorbid disorders or conditions selected from the disorders and conditions enumerated in claim 1.
31. A method according to aspect 30, wherein the compound of formula I is administered to a human for the treatment of major depressive disorder and concomitant generalised anxiety disorder.
35. A method according to aspect 1, wherein the disorder or condition being treated is fibromyalgia.
36. A method according to aspect 1, wherein the disorder or condition being treated is AIDS related neuralgia.
37. A method according to aspect 1, wherein the disorder or condition being treated is schizophrenia, schizoaffective disorder, delusional disorder, substance-induced psychotic disorder, brief psychotic disorder, shared psychotic disorder, psychotic disorder due to a general medical condition, or schizophreniform disorder.
38. A method of treating a disorder or condition selected from the group consisting of sleep disorders; pervasive development disorder; rheumatoid arthritis; osteoarthritis; fibromyalgia; human immunodeficiency virus (HIV) infections; dissociative disorders such as body dysmorphic disorders; eating disorder such as anorexia and bulimia; ulcerative colitis; Crohn's disease; irritable bowel syndrome; functional abdominal pain; chronic fatigue syndrome; sudden infant death syndrome (SIDS); overactive bladder; chronic cystitis; chemotherapy induced cystitis; cough, angiotensin converting enzyme (ACE) induced cough; itch; hiccups; premenstrual syndrome: premenstrual dysphoric disorder; schizophrenia; schizoaffective disorder; delusional disorder; substance-induced psychotic disorder; brief psychotic disorder; shared psychotic disorder; psychotic disorder due to a general medical condition; schizophreniform disorder; amenorrheic disorders such as dysmenorrhea; obesity; epilepsy: movement disorders such as primary movement disorders, spasticities, Scott's syndrome, Tourette's syndrome, palsys, amyolateral sclerosis (ALS), akinetic-rigid disorders, akinesias, dyskinesias (e.g., familial paroxysmal dyskinesia, tardive dyskinesia, tremor, chorea, myoclonus, tics and other dyskinesias) restless leg syndrome and movement disorders associated with Parkinson's disease or Huntington's disease; mastalgia syndromes; motion sickness; immune dysfunctions; generalised anxiety disorder; panic disorder; phobias, including social phobia, agoraphobia, and specific phobias; obsessive-compulsive disorder; post-traumatic stress disorder; depression including major depression, single episode depression, recurrent depression, child abuse induced depression, postpartum depression and dysthemia; cyclothymia; bipolar disorder; neurocardiac disorders such as neurocardiac syncope, neurogenic syncope, hypersensitive Carotid sinus, neurovascular syndrome and arrythmias including arrythmias secondary to gastrointestinal disturbances; addiction disorders involving addictions to behaviors; HIV-1 associated dementia, AIDS dementia complex, HIV encephalopathy, HIV related neuralgias; AIDS related neuralgias; epilepsy; and attention deficit hyperactivity disorder in a mammal, including a human, comprising administering to said mammal an amount of a compound of the formula I, as defined in claim 1, that is effective in antagonizing the effect of substance P at its receptor site.
39. The present invention also relates to a method of treating a disorder or condition selected from the group consisting of pain resulting from soft tissue and peripheral damage, such as acute trauma; postherpetic neuralgia, trigeminal neuralgia, segmental or intercostal neuralgia and other neuralgias; pain associated with osteoarthritis and rheumatoid arthritis; musculo-skeletal pain, such as pain experienced after trauma; spinal pain, dental pain, myofascial pain syndromes, episiotomy pain, and pain resulting from burns; deep and visceral pain, such as heart pain, muscle pain, eye pain, orofacial pain, for example, odontalgia, abdominal pain, gynaecological pain, for example, dysmenorrhoea, labour pain and pain associated with endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, neuropathic lower back pain, HIV related neuropathic pain, diabetic neuropathic pain, and arachnoiditis; neuropathic and non-neuropathic pain associated with carcinoma, often referred to as cancer pain; central nervous system pain, such as pain due to spinal cord or brain stem damage; lower back pain; sciatica; phantom limb pain, headache, including migraine and other vascular headaches, acute or chronic tension headache, cluster headache, temperomandibular pain and maxillary sinus pain; pain resulting from ankylosing spondylitis and gout; pain caused by increased bladder contractions; post operative pain; scar pain; and chronic non-neuropathic pain such as pain associated with fibromyalgia, HIV, rheumatoid and osteoarthritis, anthralgia and myalgia, sprains, strains and trauma such as broken bones; and post surgical pain in a mammal, including a human, comprising administering to said mammal an amount of a compound of the formula I, as defined in claim 1, or a pharmaceutically acceptable salt thereof, that is effective in treating such disorder or condition.
40. A method of treating a disorder or condition selected from the group consisting of pain resulting from soft tissue and peripheral damage, such as acute trauma; postherpetic neuralgia, trigeminal neuralgia, segmental or intercostal neuralgia and other neuralgias; pain associated with osteoarthritis and rheumatoid arthritis; musculo-skeletal pain, such as pain experienced after trauma; spinal pain, dental pain, myofascial pain syndromes, episiotomy pain, and pain resulting from burns; deep and visceral pain, such as heart pain, muscle pain, eye pain, orofacial pain, for example, odontalgia, abdominal pain, gynaecological pain, for example, dysmenorrhoea, labour pain and pain associated with endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, neuropathic lower back pain, HIV related neuropathic pain, diabetic neuropathic pain, and arachnoiditis; neuropathic and non-neuropathic pain associated with carcinoma, often referred to as cancer pain; central nervous system pain, such as pain due to spinal cord or brain stem damage; lower back pain; sciatica; phantom limb pain, headache, including migraine and other vascular headaches, acute or chronic tension headache, cluster headache, temperomandibular pain and maxillary sinus pain; pain resulting from ankylosing spondylitis and gout; pain caused by increased bladder contractions; post operative pain; scar pain; and chronic non-neuropathic pain such as pain associated with fibromyalgia, HIV, rheumatoid and osteoarthritis, anthralgia and myalgia, sprains, strains and trauma such as broken bones; and post surgical pain in a mammal, including a human, comprising administering to said mammal an amount of a compound of the formula I, as defined in claim 1, or a pharmaceutically acceptable salt thereof, that is effective in antagonizing the effect of substance P at its receptor site.
41. A method according to aspect 38, wherein the disorder or condition that is being treated is neuropathic pain.
42. A method according to aspect 38, wherein the disorder or condition that is being treated is AIDS related neuralgia.
43. A method according to aspect 38, wherein the disorder or condition that is being treated is pain associated with fibromyalgia.
44. A method according to aspect 38, wherein the disorder or condition that is being treated is selected from neuropathic lower back pain, HIV related neuropathic pain, diabetic neuropathic pain, arachnoiditis and neuropathic and non-neuropathic pain associated with carcinoma.
45. A method according to aspect 1, wherein the compound of the formula I that is employed in such method is:
   (2S,3S)-3-(5-tert-butyl-2-methoxybenzyl)amino-2-(3-trifluoromethoxyphenyl)piperidine;
   (2S,3S)-3-(2-isopropoxy-5-trifluoromethoxybenzyl)amino-2-phenyl-piperidine;
   (2S,3S)-3-(2-ethoxy-5-trifluoromethoxybenzyl)amino-2-phenyl-piperidine;
   (2S,3S)-3-(2-methoxy-5-trifluoromethoxybenzyl)-amino-2-phenylpiperidine;
   (2S,3S)-3(-5-tert-butyl-2-trifluoromethoxybenzyl)amino-2-phenylpiperidine;
   2-(diphenylmethyl)-N-(2-methoxy-5-trifluoromethoxy-phenyl)methyl-1-azabicyclo[2.2.2]octan-3-amine;
   (2S,3S)-3-[5-chloro-2-(2,2,2-trifluoroethoxy)-benzyl]amino-2-phenylpiperidine;
   (2S,3S)-3-(5-tert-butyl-2-trifluoromethoxybenzyl)amino-2-phenylpiperidine;
   (2S,3S)-3-(2-isopropoxy-5-trifluoromethoxybenzyl)amino-2-phenylpiperidine;
   (2S,3S)-3-(2-difluoromethoxy-5-trifluoromethoxybenzyl)-amino-2-phenylpiperidine;
   (2S,3S)-2-phenyl-3-[2-(2,2,2-trifluoroethoxybenzyl)-aminopiperidine; or
   (2S,3S)-2-phenyl-3-(2-trifluoromethoxybenzyl)]aminopiperidine;
   or a pharmaceutically acceptable salt thereof.
46. A method according to aspect 1, wherein the compound of formula I is administered to a human for the treatment of major depressive disorder and concomitant premenstrual dysphoric disorder.
47. A method according to aspect 1, wherein the compound of formula I is administered to a human for the treatment of major depressive disorder and concomitant dysthymia.
48. A method according to aspect 1, wherein the compound of formula I is administered to a human for the treatment of major depressive disorder and concomitant fibromyalgia.
49. A method according to aspect 1, wherein the compound of formula I is administered to a human for the treatment of major depressive disorder and a concomitant somatoform disorder selected from somitization disorder, hypochondriasis, somatoform pain disorder and undifferentiated somatoform disorder.
50. A method according to aspect 1, wherein the compound of formula I is administered to a human for the treatment of generalized anxiety disorder and concomitant irritable bowel syndrome.
51. A method according to aspect 1, wherein the compound of formula I is administered to a human for the treatment of generalized anxiety disorder and concomitant functional abdominal pain.
52. A method according to aspect 1, wherein the compound of formula I is administered to a human for the treatment of generalized anxiety disorder and concomitant neuropathic pain.
53. A method according to aspect 1, wherein the compound of formula I is administered to a human for the treatment of generalized anxiety disorder and concomitant premenstrual dysphoric disorder.
54. A method according to aspect 1, wherein the compound of formula I is administered to a human for the treatment of generalized anxiety disorder and concomitant dysthymia.
55. A method according to aspect 1, wherein the compound of formula I is administered to a human for the treatment of generalized anxiety disorder and concomitant fibromyalgia.
56. A method according to aspect 1, wherein the compound of formula I is administered to a human for the treatment of generalized anxiety disorder and a concomitant somatoform disorder selected from somitization disorder, hypochondriasis, conversion disorder, body dysmorphic disorder, somatoform pain disorder and undifferentiated somatoform disorder.
57. A method according to aspect 1, wherein the compound of formula I is administered to a human for the treatment of major depressive disorder accompanied by one or more somatic symptoms selected from loss of appetite, sleep disturbances (e.g., insomnia, interrupted sleep, early morning awakening, tired awakening), loss of libido, restlessness, fatigue, constipation, dyspepsia, heart palpitations, aches and pains (e.g., headache, neck pain, back pain, limb pain, joint pain, abdominal pain), dizziness, nausea, heartburn, nervousness, tremors, burning and tingling sensations, morning stiffness, abdominal symptoms (*e*.*g*., abdominal pain, abdominal distention, gurgling, diarrhea), and the symptoms associated with generalized anxiety disorder.
58. A method according to aspect 1, wherein the compound of formula I is administered to a human for the treatment of major depressive disorder accompanied by one or more somatic symptoms selected from fatigue, headache, neck pain, back pain, limb pain, joint pain, abdominal pain, abdominal distention, gurgling, diarrhea nervousness, and the symptoms associated with generalized anxiety disorder.
59. A method according to aspect 1, wherein the compound of formula I is administered to a human for the treatment of generalized anxiety disorder accompanied by one or more somatic symptoms selected from loss of appetite, sleep disturbances (e.g., insomnia, interrupted sleep, early morning awakening, tired awakening), loss of libido, restlessness, fatigue, constipation, dyspepsia, heart palpitations, aches and pains (e.g., headache, neck pain, back pain, limb pain, joint pain, abdominal pain), dizziness, nausea, heartburn, nervousness, tremors, burning and tingling sensations, morning stiffness, abdominal symptoms (e.g., abdominal pain, abdominal distention, gurgling, diarrhea), and the symptoms associated with major depressive disorder.
60. A method according to aspect 1, wherein the compound of formula I is administered to a human for the treatment of generalized anxiety disorder accompanied by one or more somatic symptoms selected from fatigue, headache, neck pain, back pain, limb pain, joint pain, abdominal pain, abdominal distention, gurgling, diarrhea nervousness, and the symptoms associated with major depressive disorder.
61. A method according to aspect 11, wherein the mammal being treated is a human who has not exhibited an adequate treatment response following treatment for the same disorder or condition with a selective serotonin reuptake inhibitor.
62. A method according to aspect 12, wherein the mammal being treated is a human who has not exhibited an adequate treatment response following treatment for the same disorder or condition with a selective serotonin reuptake inhibitor.
63. A method according to aspect 18, wherein the mammal being treated is a human who has not exhibited an adequate treatment response following treatment for the same disorder or condition with a selective serotonin reuptake inhibitor.
64. A method according to aspect 21, wherein the mammal being treated is a human who has not exhibited an adequate treatment response following treatment for the same disorder or condition with a selective serotonin reuptake inhibitor.
65. A method according to aspect 25, wherein the mammal being treated is a human who has not exhibited an adequate treatment response following treatment for the same disorder or condition with a selective serotonin reuptake inhibitor.
66. A method according to aspect 1, wherein the disorder or condition being treated is selected from HIV-1 associated dementia, AIDS dementia complex, HIV encephalopathy, and HIV related neuralgias.
67. A method according to aspect 1, wherein a Group A compound is employed in such method.
68. A method according to aspect 67, wherein the Group A compound that is employed in'such method is selected from:
   (2S,3S)-3-(6-methoxy-1-methyl-1-trifluoromethylisochroman-7-yl)methylamino-2-phenylpiperidine;
   (2S,3S)-3-[(1R)-6-methoxy-1-methyl-1-trifluoromethylisochroman-7-yl]methylamino-2-phenylpiperidine;
   (2S,3S)-N-(5-isopropyl-2-methoxyphenyl)methyl-2-di-phenylmethyl-1-azabicyclo[2.2.2]-octan-3-amine; and
   (2S,3S)-N-(5-tert-butyl-2-methoxyphenyl)-methyl-2-diphenylmethyl-1-azabicyclo[2.22]-octan-3-amine;
   and the pharmaceutically acceptable salts of such compounds.

## Claims

1. The use of a compound of formula I 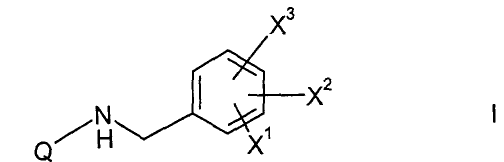 wherein X¹ is hydrogen, (C₁-C₁₀) alkoxy optionally substituted with from one to three fluorine atoms or (C₁-C₁₀) alkyl optionally substituted with from one to three fluorine atoms;
X² and X³ are independently selected from halo, hydrogen, nitro, (C₁-C₁₀) alkyl optionally substituted with from one to three fluorine atoms, (C₁-C₁₀) alkoxy optionally substituted with from one to three fluorine atoms, trifluoromethyl, hydroxy, phenyl, cyano, amino, (C₁-C₆)-alkylamino, di-(C₁-C₆)alkylamino, -C(=O)-NH-(C₁-C₆)alkyl, (C₁-C₆)alkyl-C(=O)-NH-(C₁-C₆)alkyl, hydroxy(C₁-C₄)alkyl, (C₁-C₄)alkoxy(C₁-C₄)alkyl, -NHC(=O)H and -NHC(=O)-(C₁-C₆)alkyl; and
Q is a group of the formula 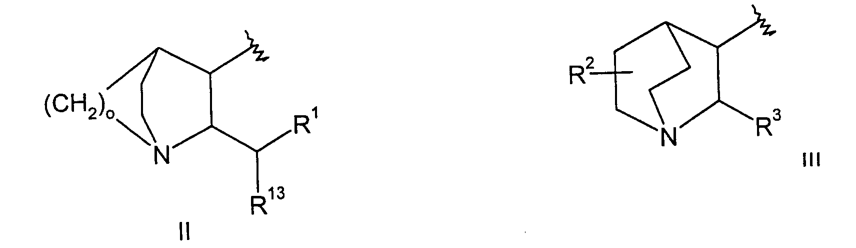 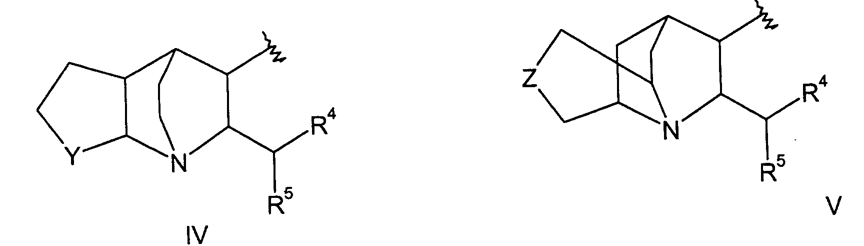 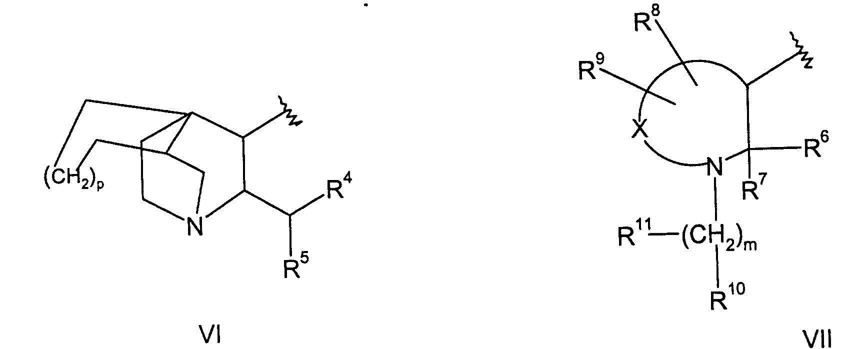 OR 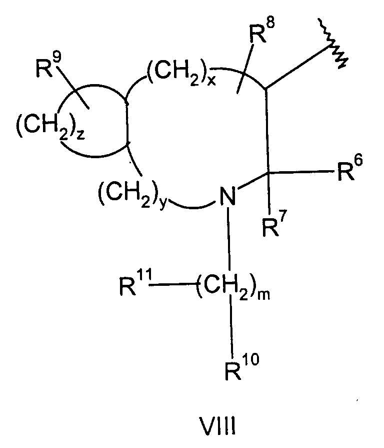 wherein R¹ is a radical selected from furyl, thienyl, pyridyl, indolyl, biphenyl and phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, benzyloxycarbonyl and (C₁-C₃) alkoxy-carbonyl;
R¹³ is selected from (C₃-C₄) branched alkyl, (C₅-C₆) branched alkenyl, (C₅-C₇) cycloalkyl, and the radicals named in the definition of R¹;
R² is hydrogen or (C₁-C₆) alkyl;
R³ is phenyl, biphenyl, naphthyl, pyridyl, benzhydryl, thienyl or furyl, and R³ may optionally be substituted with from one to three substituents independently selected from halo, (C₁-C₁₀) alkyl optionally substituted with from one to three fluorine atoms and (C₁-C₁₀) alkoxy optionally substituted with from one to three fluorine atoms;
Y is (CH₂)ₗ wherein I is an integer from one to three, or Y is a group of the formula 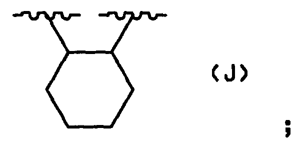
Z is oxygen, sulfur, amino, (C₁-C₃)alkylamino or (CH₂)ₙ wherein n is zero, one or two;
o is two or three;
p is zero or one;
x is an integer from zero to four;
y is an integer from zero to four;
z is an integer from one to six, and the ring in formula VIII containing (CH₂)_{z} may contain from zero to three double bonds, and one of the carbons of said (CH₂)_{z} may optionally be replaced by oxygen, sulphur or nitrogen;
R⁴ is furyl, thienyl, pyridyl, indolyl, biphenyl, or phenyl optionally substituted with one or two substituents independently selected from halo, (C₁-C₁₀) alkyl optionally substituted with from one to three fluorine atoms, (C₁-C₁₀) alkoxy optionally substituted with from one to three fluorine atoms, carboxy, (C₁-C₃) alkoxy-carbonyl and benzyloxycarbonyl;
R⁵ is thienyl, biphenyl or phenyl optionally substituted with one or two substituents independently selected from halo, (C₁-C₁₀) alkyl optionally substituted with from one to three fluorine atoms and (C₁-C₁₀) alkoxy optionally substituted with from one to three fluorine atoms;
X is (CH₂)_{q} wherein q is an integer from 1 to 6, and wherein any one of the carbon-carbon single bonds in said (CH₂)_{q} may optionally be replaced by a carbon-carbon double bond, and wherein any one of the carbon atoms of said (CH₂)_{q} may optionally be substituted with R⁸, and wherein any one of the carbon atoms of said (CH₂)_{q} may optionally be substituted with R⁹;
m is an integer from 0 to 8, and any one of the carbon-carbon single bonds of (CH₂)ₘ may optionally be replaced by a carbon-carbon double bond or a carbon-carbon triple bond, and any one of the carbon atoms of said (CH₂)ₘ may optionally be substituted with R¹¹;
R⁶ is a radical selected from hydrogen, (C₁-C₆) straight or branched alkyl, (C₃-C₇) cycloalkyl wherein one of the carbon atoms may optionally be replaced by nitrogen, oxygen or sulfur; aryl selected from biphenyl, phenyl, indanyl and naphthyl; heteroaryl selected from thienyl, furyl, pyridyl, thiazolyl, isothiazolyl, oxazolyl, isoxazolyl, triazolyl, tetrazolyl and quinolyl; phenyl (C₂-C₆) alkyl, benzhydryl and benzyl, wherein each of said aryl and heteroaryl groups and the phenyl moieties of said benzyl, phenyl (C₂-C₆) alkyl and benzhydryl may optionally be substituted with one or more substituents independently selected from halo, nitro, (C₁-C₁₀) alkyl optionally substituted with from one to three fluorine atoms, (C₁-C₁₀) alkoxy optionally substituted with from one to three fluorine atoms, amino, hydroxy-(C₁-C₆)alkyl, (C₁-C₆)alkoxy-(C₁-C₆)alkyl, (C₁-C₆)-alkylamino, (C₁-C₆)alkyl-O-C(=O)-, (C₁-C₆) alkyl-O-C(=O)- (C₁-C₆)alkyl, (C₁-C₆)alkyl-C(=O)-O-, (C₁-C₆)alkyl-C(=O)- (C₁-C₆)alkyl-O-, (C₁-C₆)alkyl-C(=O)-, (C₁-C₆)alkyl-C(=O)- (C₁-C₆)alkyl-, di-(C₁-C₆)alkylamino, -C(=O)NH-(C₁-C₆)alkyl, (C₁-C₆)-alkyl-C(=O)-NH-(C₁-C₆)alkyl, -NHC(=O)H and
-NHC(=O)-(C₁-C₆) alkyl; and wherein one of the phenyl moieties of said benzhydryl may optionally be replaced by naphthyl, thienyl, furyl or pyridyl;
R⁷ is hydrogen, phenyl or (C₁-C₆)alkyl;
or R⁶ and R⁷, together with the carbon to which they are attached, form a saturated carbocyclic ring having from 3 to 7 carbon atoms wherein one of said carbon atoms may optionally be replaced by oxygen, nitrogen or sulfur;
R⁸ and R⁹ are each independently selected from hydrogen, hydroxy, halo, amino, oxo (=O), nitrile, hydroxy-(C₁-C₆)-alkyl, (C₁-C₆)alkoxy-(C₁-C₆)alkyl, (C₁-C₆)alkylamino, di-(C₁-C₆)alkylamino, (C₁-C₆)alkoxy, (C₁-C₆)alkyl-O-C(=O)-, (C₁-C₆)alkyl-O-C(=O)-(C₁-C₆)alkyl, (C₁-C₆)alkyl-C(=O)-O-, (C₁-C₆)alkyl-C(=O)-(C₁-C₆)alkyl-O-, (C₁-C₆)alkyl-C-, (C₁-C₆)alkyl-C(=O)-(C₁-C₆)alkyl-, and the radicals set forth in the definition of R⁶;
R¹⁰ is NHC(=O)R¹², NHCH₂R¹², NHSO₂R¹² or one of the radicals set forth in any of the definitions of R⁶, R⁸ and R⁹;
R¹¹ is oximino (=NOH) or one of the radicals set forth in any of the definitions of R⁶, R⁸ and R⁹; and
R¹² is (C₁-C₆)alkyl, hydrogen, phenyl(C₁-C₆)alkyl or phenyl optionally substituted with (C₁-C₆)alkyl;
with the proviso that (a) when m is 0, R¹¹ is absent, (b) neither R⁸, R⁹, R¹⁰ nor R¹¹ can form, together with the carbon to which it is attached, a ring with R⁷, (c) when Q is a group of the formula VIII, R⁸ and R⁹ cannot be attached to the same carbon atom, (d) when R⁸ and R⁹ are attached to the same carbon atom, then either each of R⁸ and R⁹ is independently selected from hydrogen, fluoro, (C₁-C₆) alkyl, hydroxy-(C₁-C₆)alkyl and (C₁-C₆)alkoxy-(C₁-C₆)alkyl, or R⁸ and R⁹, together with the carbon to which they are attached, form a (C₃-C₆) saturated carbocyclic ring that forms a spiro compound with the nitrogen-containing ring to which they are attached, (e) when neither X¹, X² nor X³ is a fluorinated alkoxy group, at least one of R¹, R³, R⁴, R⁵, R⁶, R⁷ and R¹³ is an aryl group substituted with a fluorinated alkoxy group;
or a pharmaceutically acceptable salt thereof,
or a compound selected from the group consisting of:
(2S,3S)-3-(6-methoxy-3-trifluoromethyl-1,3-dihydroisobenzofuran-5-yl)methylamino-2-phenylpiperidine;
(2S,3S)-3-(6-methoxy-1-methyl-1-trifluoromethylisochroman-7-yl)methylamino-2-phenylpiperidine;
(2S,3S)-3-(6-methoxy-3-methyl-3-trifluoromethyl-1,3-dihydroisobenzofuran-5-yl)methylamino-2-phenylpiperidine;
(2S,3S)-3-(6-methoxy-3-phenyl-3-trifluoromethyl-1,3-dihydroisobenzofuran-5-yl)methylamino-2-phenylpiperidine;
(2S,3S)-3-[1-(6-methoxy-3-methyl-3-trifluoromethyl-1,3-dihydroisobenzofuran-5-yl)ethylamino]-2-phenylpiperidine;
(2S,3S)-3-[(1R)-6-methoxy-1-methyl-1-trifluoromethylisochroman-7-yl]methylamino-2-phenylpiperidine;
(2S,3S)-3-[(3R)-6-methoxy-3-methyl-3-trifluoromethyl-1,3-dihydroisobenzofuran-5-yl)methylamino-2-phenylpiperidine;
(2S,3S)-N-(5-ethyl-2-methoxyphenyl)methyl-2-diphenylmethyl-1-azabi-cyclo[2.2.2]-octan-3-amine;
(2S,3S)-N-(5-isopropyl-2-methoxyphenyl)methyl-2-di-phenylmethyl-1-azabicyclo[2.2.2]-octan-3-amine;
(2S,3S)-N-(5-sec-butyl-2-methoxyphenyl)-methyl-2-diphenylmethyl-1-azabicyclo[2.2.2]-octan-3-amine;
(2S,3S)-N-(5-tert-butyl-2-methoxyphenyl)-methyl-2-diphenylmethyl-1-azabicyclo[2.2.2]-octan-3-amine; and
(2S,3S)-N-(5-methyl-2-methoxyphenyl)methyl-2-diphenylmethyl-1-azabicyclo[2.2.2]-octan-3-amine;
or a pharmaceutically acceptable salt thereof, in the manufacture of a medicament for treating a disorder or condition selected from sleep disorders; autism; pervasive development disorder; rheumatoid arthritis; osteoarthritis; fibromyalgia; human immunodeficiency virus (HIV) infections; dissociative disorders such as body dysmorphic disorders; eating disorder such as anorexia and bulimia; ulcerative colitis; Crohn's disease; irritable bowel syndrome; functional abdominal pain; chronic fatigue syndrome; sudden infant, death syndrome (SIDS); overactive bladder; chronic cystitis; chemotherapy induced cystitis; cough, angiotensin converting enzyme (ACE) induced cough; itch; hiccups; premenstrual syndrome: premenstrual dysphoric disorder; schizophrenia; schizoaffective disorder; delusional disorder; substance-induced psychotic disorder; brief psychotic disorder; shared psychotic disorder; psychotic disorder due to a general medical condition; schizophreniform disorder; amenorrheic disorders such as dysmenorrhea; obesity; epilepsy: movement disorders such as primary movement disorders, spasticities, Scott's syndrome, Tourette's syndrome, palsys, amyolateral sclerosis (ALS), akinetic-rigid disorders, akinesias, dyskinesias, restless leg syndrome and movement disorders associated with Parkinson's disease or Huntington's disease; mastalgia syndromes; motion sickness; immune dysfunctions; generalized anxiety disorder; panic disorder; phobias, including social phobia, agoraphobia, and specific phobias; obsessive-compulsive disorder; post-traumatic stress disorder; depression including major depression, single episode depression, recurrent depression, child abuse induced depression, postpartum depression and dysthemia; cyclothymia; bipolar disorder; neurocardiac disorders such as neurocardiac syncope, neurogenic syncope, hypersensitive Carotid sinus, neurovascular syndrome and arrythmias including arrythmias secondary to gastrointestinal disturbances; addiction disorders involving addictions to behaviors; HIV-1 associated dementia, AIDS dementia complex, HIV encephalopathy, HIV related neuralgias; AIDS related neuralgias; epilepsy; and attention deficit hyperactivity disorder in a mammal.

2. The use according to claim 1, wherein the compound of formula I that is employed in such method is selected from the following compounds and their pharmaceutically acceptable salts:
2-(diphenylmethyl)-N-((2-difluoromethoxy)-phenyl)methyl-1-azabicyclo[2.2.2]octan-3-amine;
(2S,3S)-N-(2-methoxy-5-trifluoromethoxy-phenyl)methyl-2-diphenylmethyl-1-azabicyclo[2.2.2]octane-3-amine;
(2S,3S)-2-phenyl-3-[2-(2,2,2-trifluoroethoxy)-benzyl]aminopiperidine;
(2S,3S)-3-(2-methoxy-5-trifluoromethoxybenzyl)-amino-2-phenylpiperidine;
(2S,3S)-3-(2-hydroxy-5-trifluoromethoxybenzyl)-amino-2-phenylpiperidine;
(2S,3S)-2-phenyl-3-(3-trifluoromethoxybenzyl)-aminopiperidine;
(2S,3S)-1-(5,6-dimethoxyhexyl)-3-(2-methoxy-5-trifluoromethoxybenzyl)amino-2-phenylpiperidine;
(2S,3S)-2-phenyl-3-(2-trifluoromethoxybenzyl)-aminopiperidine;
(2S,3S)-3-[5-chloro-2-(2,2,2-trifluoroethoxy)-benzyl]amino-2-phenylpiperidine;
(2S,3S)-3-(5-t-butyl-2-trifluoromethoxy-benzyl)amino-2-phenylpiperidine;
3-(5-tert-butyl-2-methoxybenzyl)amino-2-(3-trifluoromethoxyphenyl)piperidine;
3-(2-isopropoxy-5-trifluoromethoxybenzyl)amino-2-phenyl)piperidine; and
3-(2-difluoromethoxy-5-trifluoromethoxybenzyl)-amino-2-phenylpiperidine.

3. The use according to claim 1, wherein the disorder or condition being treated is selected from cyclothymia or bipolar disorder, an addiction to a behavior, a sleep disorder, premenstrual syndrome or premenstrual dysphoric disorder, autism or pervasive development disorder, Scott's syndrome or Tourette's syndrome, obsessive-compulsive disorder,
or movement disorders such as primary movement disorders, spasticities, Scott's syndrome, Tourette's syndrome, palsys, amyolateral sclerosis (ALS), akinetic-rigid disorders, akinesias, dyskinesias, restless leg syndrome and movement disorders associated with Parkinson's disease or Huntington's disease,
or major depressive disorder, generalized anxiety disorder, irritable bowel syndrome, functional abdominal pain, an HIV infection, an immune dysfunction, a disorder or condition selected from neurocardiac disorders such as neurocardiac syncope, neurogenic syncope, hypersensitive Carotid sinus, neurovascular syndrome and arrythmias including arrythmias secondary to gastrointestinal disturbances,
or the disorder or condition is selected from major depression, single episode depression, recurrent depression, child abuse induced depression, postpartum depression, dysthymia, cyclothymia and bipolar disorder,
or the disorder or condition is selected from as body dysmorphic disorders and eating disorders such as anorexia and bulimia,
or the disorder or condition is selected from schizophrenia, schizoaffective disorder, delusional disorder, substance-induced psychotic disorder, brief psychotic disorder, shared psychotic disorder, psychotic disorder due to a general medical condition, and schizophreniform disorder,
or the disorder or condition is selected from premenstrual syndrome, premenstrual dysphoric disorder, and amenorrheic disorders such as dysmenorrhea,
or the disorder or condition is selected from Crohn's disease, ulcerative colitis, irritable bowel syndrome and functional abdominal pain,
or the disorder or condition is selected from autism, pervasive development disorder, and attention deficit hyperactivity disorder,
or the disorder or condition is selected from chronic fatigue syndrome, sudden infant death syndrome (SIDS), obesity, and epilepsy,
or the disorder or condition is selected from generalized anxiety disorder, panic disorder, obsessive-compulsive disorder, post-traumatic stress disorder, and phobias, including social phobia, agoraphobia, and specific phobias,
or the disorder or condition is selected from cough, angiotensin converting enzyme (ACE) induced cough, itch, and hiccups,
or the disorder or condition is selected from overactive bladder; chronic cystitis and chemotherapy induced cystitis, or a sleep disorder.

4. The use according to claim 1, wherein the disorder is a major depressive disorder with concomitant neuropathic pain.

5. The use according to claim 1, wherein the disorder is any two or more comorbid disorders or conditions selected from the disorders and conditions enumerated in claim 1.

6. The use according to claim 5, wherein the disorder is major depressive disorder and concomitant generalized anxiety disorder.

7. The use according to claim 1, wherein the disorder or condition is fibromyalgia, AIDS related neuralgia, schizophrenia, schizoaffective disorder, delusional disorder, substance-induced psychotic disorder, brief psychotic disorder, shared psychotic disorder, psychotic disorder due to a general medical condition, schizophreniform disorder,
sleep disorders; pervasive development disorder; rheumatoid arthritis; osteoarthritis; fibromyalgia; human immunodeficiency virus (HIV) infections; dissociative disorders such as body dysmorphic disorders; eating disorder such as anorexia and bulimia; ulcerative colitis; Crohn's disease; irritable bowel syndrome; functional abdominal pain; chronic fatigue syndrome; sudden infant death syndrome (SIDS); overactive bladder; chronic cystitis; chemotherapy induced cystitis; cough, angiotensin converting enzyme (ACE) induced cough; itch; hiccups; premenstrual syndrome: premenstrual dysphoric disorder; schizophrenia; schizoaffective disorder; delusional disorder; substance-induced psychotic disorder; brief psychotic disorder; shared psychotic disorder; psychotic disorder due to a general medical condition; schizophreniform disorder; amenorrheic disorders such as dysmenorrhea; obesity; epilepsy: movement disorders such as primary movement disorders, spasticities, Scott's syndrome, Tourette's syndrome, palsys, amyolateral sclerosis (ALS), akinetic-rigid disorders, akinesias, dyskinesias (e.g., familial paroxysmal dyskinesia, tardive dyskinesia, tremor, chorea, myoclonus, tics and other dyskinesias) restless leg syndrome and movement disorders associated with Parkinson's disease or Huntington's disease; mastalgia syndromes; motion sickness; immune dysfunctions; generalized anxiety disorder; panic disorder; phobias, including social phobia, agoraphobia, and specific phobias; obsessive-compulsive disorder; post-traumatic stress disorder; depression including major depression, single episode depression, recurrent depression, child abuse induced depression, postpartum depression and dysthemia; cyclothymia; bipolar disorder; neurocardiac disorders such as neurocardiac syncope, neurogenic syncope, hypersensitive Carotid sinus, neurovascular syndrome and arrythmias including arrythmias secondary to gastrointestinal disturbances; addiction disorders involving addictions to behaviors; HIV-1 associated dementia, AIDS dementia complex, HIV encephalopathy, HIV related neuralgias; AIDS related neuralgias; epilepsy; attention deficit hyperactivity disorder,
or pain resulting from soft tissue and peripheral damage, such as acute trauma; postherpetic neuralgia, trigeminal neuralgia, segmental or intercostal neuralgia and other neuralgias; pain associated with osteoarthritis and rheumatoid arthritis; musculo-skeletal pain, such as pain experienced after trauma; spinal pain, dental pain, myofascial pain syndromes, episiotomy pain, and pain resulting from burns; deep and visceral pain, such as heart pain, muscle pain, eye pain, orofacial pain, for example, odontalgia, abdominal pain, gynaecological pain, for example, dysmenorrhoea, labour pain and pain associated with endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, neuropathic lower back pain, HIV related neuropathic pain, diabetic neuropathic pain, and arachnoiditis; neuropathic and non-neuropathic pain associated with carcinoma, often referred to as cancer pain; central nervous system pain, such as pain due to spinal cord or brain stem damage; lower back pain; sciatica; phantom limb pain, headache, including migraine and other vascular headaches, acute or chronic tension headache, cluster headache, temperomandibular pain and maxillary sinus pain; pain resulting from ankylosing spondylitis and gout; pain caused by increased bladder contractions; post operative pain; scar pain; and chronic non-neuropathic pain such as pain associated with fibromyalgia, HIV, rheumatoid and osteoarthritis, anthralgia and myalgia, sprains, strains and trauma such as broken bones; post surgical pain,
or pain resulting from soft tissue and peripheral damage, such as acute trauma; postherpetic neuralgia, trigeminal neuralgia, segmental or intercostal neuralgia and other neuralgias; pain associated with osteoarthritis and rheumatoid arthritis; musculo-skeletal pain, such as pain experienced after trauma; spinal pain, dental pain, myofascial pain syndromes, episiotomy pain, and pain resulting from burns; deep and visceral pain, such as heart pain, muscle pain, eye pain, orofacial pain, for example, odontalgia, abdominal pain, gynaecological pain, for example, dysmenorrhoea, labour pain and pain associated with endometriosis; pain associated with nerve and root damage, such as pain associated with peripheral nerve disorders, for example, nerve entrapment and brachial plexus avulsions, amputation, peripheral neuropathies, tic douloureux, atypical facial pain, nerve root damage, neuropathic lower back pain, HIV related neuropathic pain, diabetic neuropathic pain, and arachnoiditis; neuropathic and non-neuropathic pain associated with carcinoma, often referred to as cancer pain; central nervous system pain, such as pain due to spinal cord or brain stem damage; lower back pain; sciatica; phantom limb pain, headache, including migraine and other vascular headaches, acute or chronic tension headache, cluster headache, temperomandibular pain and maxillary sinus pain; pain resulting from ankylosing spondylitis and gout; pain caused by increased bladder contractions; post operative pain; scar pain; and chronic non-neuropathic pain such as pain associated with fibromyalgia, HIV, rheumatoid and osteoarthritis, anthralgia and myalgia, sprains, strains and trauma such as broken bones; post surgical pain,
or neuropathic pain, AIDS related neuralgia, pain associated with fibromyalgia, neuropathic lower back pain, HIV related neuropathic pain, diabetic neuropathic pain, arachnoiditis and
neuropathic and non-neuropathic pain associated with carcinoma.

8. The use according to claim 1, wherein the compound of the formula I that is employed in such method is:
(2S,3S)-3-(5-tert-butyl-2-methoxybenzyl)amino-2-(3-trifluoromethoxyphenyl)piperidine;
(2S,3S)-3-(2-isopropoxy-5-trifluoromethoxybenzyl)amino-2-phenyl-piperidine;
(2S,3S)-3-(2-ethoxy-5-trifluoromethoxybenzyl)amino-2-phenyl-piperidine;
(2S,3S)-3-(2-methoxy-5-trifluoromethoxybenzyl)-amino-2-phenylpiperidine;
(2S,3S)-3(-5-tert-butyl-2-trifluoromethoxybenzyl)amino-2-phenylpiperidine;
2-(diphenylmethyl)-N-(2-methoxy-5-trifluoromethoxy-phenyl)methyl-1-azabicyclo[2.2.2]octan-3-amine;
(2S,3S)-3-[5-chloro-2-(2,2,2-trifluoroethoxy)-benzyl]amino-2-phenylpiperidine;
(2S,3S)-3-(5-tert-butyl-2-trifluoromethoxybenzyl)amino-2-phenylpiperidine;
(2S,3S)-3-(2-isopropoxy-5-trifluoromethoxybenzyl)amino-2-phenylpiperidine;
(2S,3S)-3-(2-difluoromethoxy-5-trifluoromethoxybenzyl)-amino-2-phenylpiperidine;
(2S,3S)-2-phenyl-3-[2-(2,2,2-trifluoroethoxybenzyl)-aminopiperidine; or
(2S,3S)-2-phenyl-3-(2-trifluoromethoxybenzyl)]aminopiperidine;
or a pharmaceutically acceptable salt thereof.

9. The use according to claim 1, wherein the disorder is major depressive disorder and concomitant premenstrual dysphoric disorder, major depressive disorder and concomitant dysthymia, major depressive disorder and concomitant fibromyalgia, major depressive disorder and a concomitant somatoform disorder selected from somitization disorder, hypochondriasis, somatoform pain disorder and undifferentiated somatoform disorder,
or the disorder is generalized anxiety disorder and concomitant irritable bowel syndrome, generalized anxiety disorder and concomitant functional abdominal pain, generalized anxiety disorder and concomitant neuropathic pain, generalized anxiety disorder and concomitant premenstrual dysphoric disorder, generalized anxiety disorder and concomitant dysthymia, generalized anxiety disorder and concomitant fibromyalgia, generalized anxiety disorder and a concomitant somatoform disorder selected from somitization disorder, hypochondriasis, conversion disorder, body dysmorphic disorder, somatoform pain disorder and undifferentiated somatoform disorder,
or the disorder is major depressive disorder accompanied by one or more somatic symptoms selected from loss of appetite, sleep disturbances (e.g., insomnia, interrupted sleep, early morning awakening, tired awakening), loss of libido, restlessness, fatigue, constipation, dyspepsia, heart palpitations, aches and pains (e.g., headache, neck pain, back pain, limb pain, joint pain, abdominal pain), dizziness, nausea, heartburn, nervousness, tremors, burning and tingling sensations, morning stiffness, abdominal symptoms (e.g., abdominal pain, abdominal distention, gurgling, diarrhea), and the symptoms associated with generalized anxiety disorder,
or the disorder is major depressive disorder accompanied by one or more somatic symptoms selected from fatigue, headache, neck pain, back pain, limb pain, joint pain, abdominal pain, abdominal distention, gurgling, diarrhea nervousness, and the symptoms associated with generalized anxiety disorder,
or the disorder is generalized anxiety disorder accompanied by one or more somatic symptoms selected from loss of appetite, sleep disturbances (e.g., insomnia, interrupted sleep, early morning awakening, tired awakening), loss of libido, restlessness, fatigue, constipation, dyspepsia, heart palpitations, aches and pains (*e*.*g*., headache, neck pain, back pain, limb pain, joint pain, abdominal pain), dizziness, nausea, heartburn, nervousness, tremors, burning and tingling sensations, morning stiffness, abdominal symptoms (e.g., abdominal pain, abdominal distention, gurgling, diarrhea), and the symptoms associated with major depressive disorder,
or the disorder is generalized anxiety disorder accompanied by one or more somatic symptoms selected from fatigue, headache, neck pain, back pain, limb pain, joint pain, abdominal pain, abdominal distention, gurgling, diarrhea nervousness, and the symptoms associated with major depressive disorder.

10. The use according to claim 1, wherein the disorder being treated is a major depressive disorder and the mammal being treated is a human who has not exhibited an adequate treatment response following treatment for the same disorder or condition with a selective serotonin reuptake inhibitor.

11. The use according to claim 1, wherein the disorder is generalized anxiety disorder and the mammal being treated is a human who has not exhibited an adequate treatment response following treatment for the same disorder or condition with a selective serotonin reuptake inhibitor.

12. The use according to claim 1, wherein the disorder or condition is selected from major depression, single episode depression, recurrent depression, child abuse induced depression, postpartum depression, dysthymia, cyclothymia and bipolar disorder and the mammal being treated is a human who has not exhibited an adequate treatment response following treatment for the same disorder or condition with a selective serotonin reuptake inhibitor.

13. The use according to claim 1, wherein the disorder or condition is selected from premenstrual syndrome, premenstrual dysphoric disorder, and amenorrheic disorders such as dysmenorrhea, and the mammal being treated is a human who has not exhibited an adequate treatment response following treatment for the same disorder or condition with a selective serotonin reuptake inhibitor.

14. The use according to claim 1, wherein the disorder or condition is selected from generalised anxiety disorder, panic disorder, obsessive-compulsive disorder, post-traumatic stress disorder, and phobias, including social phobia, agoraphobia, and specific phobias, and the mammal being treated is a human who has not exhibited an adequate treatment response following treatment for the same disorder or condition with a selective serotonin reuptake inhibitor.

15. The use according to claim 1, wherein the disorder or condition is selected from HIV-1 associated dementia, AIDS dementia complex, HIV encephalopathy, and HIV related neuralgias.

16. The use according to claim 1, wherein a Group A compound is employed.

17. The use according to claim 16, wherein the Group A compound that is employed is selected from:
(2S,3S)-3-(6-methoxy-1-methyl-1-trifluoromethylisochroman-7-yl)methylamino-2-phenylpiperidine;
(2S,3S)-3-[(1R)-6-methoxy-1-methyl-1-trifluoromethylisochroman-7-yl]methylamino-2-phenylpiperidine;
(2S,3S)-N-(5-isopropyl-2-methoxyphenyl)methyl-2-di-phenylmethyl-1-azabicyclo[2.2.2]-octan-3-amine; and
(2S,3S)-N-(5-tert-butyl-2-methoxyphenyl)-methyl-2-diphenylmethyl-1-azabicyclo[2.2.2]-octan-3-amine;
and the pharmaceutically acceptable salts of such compounds.
